(19) Europäisches Patentamt
European Patent Office
Office européen des brevets

(11) Veröffentlichungsnummer: **0 003 771**
**B1**

(12) # EUROPÄISCHE PATENTSCHRIFT

(45) Veröffentlichungstag der Patentschrift: **20.10.82**

(51) Int. Cl.³: **C 07 D 215/22**, A 61 K 31/47, C 07 D 401/12

(21) Anmeldenummer: **79100348.6**

(22) Anmeldetag: **07.02.79**

(54) **Neue Carbostyrilderivate, ihre Herstellung und diese Verbindungen enthaltende Arzneimittel.**

(30) Priorität: **17.02.78 DE 2806721**
**09.12.78 DE 2853314**

(43) Veröffentlichungstag der Anmeldung: **05.09.79 Patentblatt 79/18**

(45) Bekanntmachung des Hinweises auf die Patenterteilung: **20.10.82 Patentblatt 82/42**

(84) Benannte Vertragsstaaten: **BE CH DE FR GB IT LU NL SE**

(56) Entgegenhaltungen:
**DE-A-2 453 113**
**DE-A-2 502 156**
**DE-A-2 527 937**
**DE-A-2 825 048**
**JP-A-21 017 678**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 720, D-7950 Biberach (Riss) (DE)**

(72) Erfinder: **Müller, Erich, Dr. Dipl.-Chem., TalfeldstraBe 34, D-7950 Biberach 1 (DE)**
Erfinder: **Nickl, Josef, Dr. Dipl.-Chem., SilcherstraBe 8, D-7950 Biberach 1 (DE)**
Erfinder: **Roch, Josef, Dr. Dipl.-Chem., Stecherweg 19, D-7950 Biberach 1 (DE)**
Erfinder: **Narr, Berthold, Dr. Dipl.-Chem., Obere Au 5, D-7950 Biberach 1 (DE)**
Erfinder: **Haarmann, Walter, Dr., 7950 Biberach 1, Schlierholzweg 8 (DE)**
Erfinder: **Weisenberger, Johannes Maximilian, Dr. Dipl.-Chem, 7950 Biberach 1, Haydnweg 5 (DE)**

EP 0 003 771 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Neue Carbostyrilderivate, ihre Herstellung und diese Verbindungen enthaltende Arzneimittel

Aus der DE-A-2 453 113 ist bekannt, dass 5-(2-Hydroxy-3-tert.-butylamino-propoxy)-3,4-dihydrocarbostyril antithrombotische Eigenschaften aufweist. Ferner werden in der DE-A-2 527 937 Hydroxycarbonylalkoxy-carbostyrile und deren Ester beschrieben, welche ebenfalls antithrombotische Eigenschaften aufweisen.

Des weiteren werden in der Japanischen Offenlegungsschrift 21 176/78 vom 27. Februar 1978 bereits Carbostyrilderivate beschrieben, welche durch eine 3-(Äthylthio)-propoxy-, 3-(Äthylsulfonyl)-propoxy- oder 2-(n-Butylthio)-äthoxygruppe substituiert sind, und ebenfalls antithrombotische Eigenschaften aufweisen.

Es wurde nun gefunden, dass die neuen Carbostyrilderivate der allgemeinen Formel

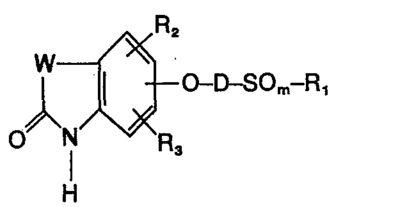

(I),

neben einer zusätzlichen positiv inotropen Wirkung insbesondere antithrombotische bzw. überlegene antithrombotische Eigenschaften aufweisen.

Gegenstand der vorliegenden Erfindung sind somit die neuen Carbostyrilderivate der obigen allgemeinen Formel I, die sie enthaltende Arzneimittel und Verfahren zu ihrer Herstellung.

In der obigen allgemeinen Formel I bedeutet

W eine gegebenenfalls durch eine Methylgruppe substituierte Vinylengruppe oder die Äthylengruppe,

m die Zahl 0, 1 oder 2,

D eine geradkettige oder verzweigte Alkylengruppe mit 2 bis 6 Kohlenstoffatomen, wobei zwischen dem Sauerstoffatom und der $SO_m$-Gruppe mindestens 2 Kohlenstoffatome liegen müssen, oder eine Xylylengruppe,

$R_1$ eine Cycloalkylgruppe mit 3 bis 6 Kohlenstoffatomen, eine Arylgruppe mit 6 bis 10 Kohlenstoffatomen oder eine Aralkylgruppe mit 7 bis 11 Kohlenstoffatomen, wobei die oben aufgeführten aromatischen Kerne durch eine Alkylgruppe mit 1 bis 4 Kohlenstoffatomen, durch eine Hydroxy-, Methoxy-, Amino-, Acetylamino-, Nitro-, Carboxyl-, Cyclohexyl-, Phenylgruppe oder ein Halogenatom monosubstituiert und zusätzlich die oben erwähnten monosubstituierten Phenylgruppen durch Alkylgruppen mit 1 bis 4 Kohlenstoffatomen und/oder Halogenatome mono- oder disubstituiert sein können (wobei die Substituenten des Phenylkerns gleich oder verschieden sein können), eine N-Methyl-cyclohexylamino-carbonylmethyl-, Amino-iminomethylen-, Pyridyl-, Pyridylmethyl-, Furfuryl-, Benzimidazolyl-, Benzthiazolyl-, Pyrimidyl-, Chinolyl-, Chinazolin-4-onyl-, 4,5-Bis-(p-chlorphenyl)-oxazol-2-yl-, Pyridyl-oxid-, Triazolyl-, Methyl-pyridyl-, Methoxy-pyridyl-, Fluor-pyridyl-, Chlor-pyridyl-, Amino-pyridyl-, Acetylaminopyridyl- oder Triphenylmethylgruppe oder auch eine tert. Butylgruppe, wenn

m die Zahl 1 darstellt,

$R_2$ und $R_3$, die gleich oder verschieden sein können, Wasserstoff- oder Halogenatome, Alkylgruppen mit 1 bis 4 Kohlenstoffatomen, Amino-, Acetylamino- oder Nitrogruppen.

Unter dem bei der Definition der Reste $R_1$, $R_2$ und $R_3$ erwähnten Ausdruck «ein Halogenatom» ist insbesondere ein Fluor-, Chlor-, Brom- oder Jodatom zu verstehen; für die bei der Definition der Reste D, $R_1$, $R_2$ und $R_3$ eingangs erwähnten Bedeutungen kommt somit für

D die Bedeutung der Äthylen-, n-Propylen-, n-Butylen-, n-Pentylen-, n-Hexylen-, 1-Methyl-äthylen-, 2-Methyl-äthylen, 1-Methyl-n-propylen-, 2-Methyl-n-propylen-, 3-Methyl-n-propylen-, 1-Methyl-n-butylen-, 2-Methyl-n-butylen-, 3-Methyl-n-butylen-, 4-Methyl-n-butylen-, 1-Methyl-n-pentylen-, 2-Methyl-n-pentylen-, 3-Methyl-n-pentylen-, 4-Methyl-n-pentylen-, 5-Methyl-n-pentylen-, 1,1-Dimethyläthylen-, 1,2-Dimethyläthylen-, 2,2-Dimethyläthylen-, 1,1-Dimethyl-n-propylen-, 2,2-Dimethyl-n-propylen, 3,3-Dimethyl-n-propylen-, 1,2-Dimethyl-n-propylen-, 1,3-Dimethyl-n-propylen-, 1,1-Dimethyl-n-butylen-, 2,2-Dimethyl-n-butylen-, 3,3-Dimethyl-n-butylen-, 4,4-Dimethyl-n-butylen-, 1,2-Dimethyl-n-butylen-, 1,2-Dimethyl-n-butylen-, 1,4-Dimethyl-n-butylen-, 2,3-Dimethyl-n-butylen-, 1-Äthyläthylen-, 2-Äthyläthylen-, 1-Äthyl-n-propylen-, 2-Äthyl-n-propylen-, 3-Äthyl-n-propylen-, 1-Äthyl-n-butylen-, 2-Äthyl-n-butylen-, 3-Äthyl-n-butylen-, 4-Äthyl-n-butylen-, 1-Methyl-2-äthyl-äthylen-, 1-Methyl-2-äthyl-n-propylen-, 1-Methyl-3-äthyl-n-propylen-, 1-Methyl-2-propyl-äthylen-, 1-Propyl-äthylen-, 1-Butyl-äthylen-, 1-Propyl-n-propylen-, p-Xylylen-, o-Xylylen-, o-Xylylen- oder m-Xylylengruppe, für

$R_1$ die der Cyclopropyl-, Cyclobutyl-, Cyclopentyl-, Cyclohexyl-, Phenyl-, Benzyl-, Phenyläthyl-, Naphthyl-, Naphthylmethyl-, Cyclohexylphenyl-, Biphenyl-, Triphenylmethyl-, N-Methylcyclohexylaminocarbonylmethyl-, Amino-iminomethyl-, Pyridyl-, Pyridylmethyl-, Furfuryl-, Benzimidazolyl-, Benzthiazolyl-, Pyrimidyl-, Chinolyl-, Chinazolin-4-on-yl-, 4,5-Bis-(p-chlorphenyl)-oxazol-2-yl-, Pyridyl-oxid-, Methylphenyl-, Dimethylphenyl-, tert.-Butylphenyl-, Methyl-tert.Butylphenyl-, Methyl-pyridyl-, Methoxyphenyl-, Dimethoxyphenyl-, Methoxypyridyl-, Hydroxyphenyl-, Dihydroxyphenyl-, Fluorphenyl-, Difluorphenyl-, Trifluorphenyl-, 1,2,4-Triazolyl-, Fluor-pyridyl-, Chlorphenyl-, Dichlorphenyl-, Trichlorphenyl-, Chlorpyridyl-, Bromphenyl-, Dibromphenyl-, Aminophenyl-, Acetylaminophenyl-, Aminopyridyl-, Acetylaminopyridyl-, Nitrophenyl-, Carboxyphenyl-, Hydroxy-dichlorphenyl-, Hydroxy-dibromphenyl-, Aminodichlorphenyl-, Amino-dibromphenyl-, Hydroxy-di-tert.butylphenyl-, Methoxy-fluorphenyl-, Meth-

oxy-chlorphenyl-, Methoxy-bromphenyl-, Fluor-methylphenyl-, Chlor-methylphenyl- oder Brom-methylphenylgruppe für

$R_2$ und $R_3$, die gleich oder verschieden sein können, die des Wasserstoff-, Fluor-, Chlor-, Brom- oder Jodatoms, die der Methyl-, Äthyl-, Propyl-, Isopropyl-, Butyl-, tert.Butyl-, Nitro-, Amino- oder Acetylaminogruppe in Betracht.

Gegenstand der vorliegenden Anmeldung sind somit insbesondere diejenigen Verbindungen der allgemeinen Formel I, in der

W, D und m wie eingangs definiert sind,

$R_1$ eine Cyclohexyl-, Benzyl-, Naphthyl-, Pyridyl-, Pyrimidyl-, 1,2,4-Triazolyl-, Pyridyl-oxid-, Furfuryl-, Triphenylmethyl-, Chinolyl-, Benzimidazolyl-, Benzthiazolyl-, Chinazolin-4-on-yl-, 4,5-Bis-(p-chlorphenyl)-oxazol-2-yl-, N-Methyl-cyclohexylamino-carbonylmethyl- oder Aminoiminomethylgruppe, eine gegebenenfalls durch eine Carboxyl-, Hydroxy-, Methoxy-, Amino-, Acetylamino-, Nitro-, Cyclohexyl- oder Phenylgruppe substituierte Phenylgruppe, eine durch Halogenatome und/oder Alkylgruppen mit 1 bis 4 Kohlenstoffatomen mono- oder disubstituierte Phenylgruppe, eine durch zwei Halogenatome oder durch zwei Alkylgruppen mit 1 bis 4 Kohlenstoffatomen substituierte Hydroxyphenyl-, Halogenphenyl- oder Aminophenylgruppe,

$R_2$ ein Wasserstoff-, Chlor- oder Bromatom, die Methyl-, Amino-, Acetylamino- oder Nitrogruppe und

$R_3$ ein Wasserstoffatom bedeuten.

Bevorzugte Verbindungen der obigen allgemeinen Formel I sind jedoch diejenigen, in der

W eine gegebenenfalls durch eine Methylgruppe substituierte Vinylengruppe oder die Äthylengruppe,

m die Zahl 0, 1 oder 2,

D eine Alkylengruppe mit 2 bis 5 Kohlenstoffatomen, wobei zwischen dem Sauerstoffatom und der $SO_m$-Gruppe mindestens 2 Kohlenstoffatome liegen müssen,

$R_1$ eine Cyclohexyl-, Phenyl-, Benzyl-, Naphthyl-, Biphenyl-Cyclohexylphenyl-, Pyridyl-, Methylphenyl-, Methoxyphenyl-, Fluorphenyl-, Chlorphenyl-, Dichlorphenyl-, Trichlorphenyl-, Bromphenyl-, Dibromphenyl-, Brom-methylphenyl-, Amino-dibromphenyl- oder Hydroxy-di-tert.-butylphenylgruppe,

$R_2$ und $R_3$ je ein Wasserstoffatom bedeuten.

Ganz bevorzugte Verbindungen der obigen allgemeinen Formel I sind jedoch diejenigen, in der

W die Äthylen-, Vinylen- oder 2-Methylvinylengruppe,

m die Zahl 1 oder 2,

$R_2$ und $R_3$ je ein Wasserstoffatom,

$R_1$ die Cyclohexyl-, Phenyl-, Benzyl-, Naphthyl-(2)-, 2-Methoxyphenyl-, 4-Chlorphenyl-, 3,4-Dichlorphenyl-, 2,5-Dichlorphenyl-, 4-Amino-3,5-dibromphenyl-, 4-Hydroxy-3,5-di-tert.butylphenyl- oder Pyridyl-(2)-gruppe und

D die Äthylen-, n-Propylen- oder n-Butylengruppe bedeuten.

Erfindungsgemäss erhält man die neuen Verbindungen der allgemeinen Formel I nach folgenden Verfahren:

a) Umsetzung einer Hydroxyverbindung der allgemeinen Formel

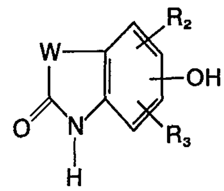

(II),

in der

$R_2$, $R_3$ und W wie eingangs definiert sind, oder deren Salze mit anorganischen oder tertiären organischen Basen mit einer Verbindung der allgemeinen Formel

$$Z - D - SO_m - R_1 \qquad (III),$$

in der

D, $R_1$ und m wie eingangs definiert sind und

Z eine nukleophil austauschbare Gruppe wie ein Halogenatom oder einen Sulfonsäureesterrest, z.B. ein Chlor-, Brom-, Jodatom, eine p-Toluolsulfonyloxy- oder Methansulfonyloxygruppe, darstellt.

Die Umsetzung wird zweckmässigerweise in einem geeigneten Lösungsmittel wie Dioxan, Tetrahydrofuran, Chloroform oder Toluol, vorzugsweise jedoch in einem wasserfreien aprotischen Lösungsmittel wie Aceton, Dimethylformamid oder Dimethylsulfoxid, gegebenenfalls in Gegenwart einer Alkalibase wie Natriumkarbonat, Kaliumcarbonat oder Natriumhydroxid bei Temperaturen zwischen 0 °C und der Siedetemperatur des verwendeten Lösungsmittels, z. B. bei Temperaturen zwischen 0 und 100 °C, vorzugsweise jedoch bei Temperaturen zwischen 10 und 50 °C, durchgeführt. Die Umsetzung kann jedoch auch ohne Lösungsmittel durchgeführt werden.

b) Zur Herstellung von Verbindungen der allgemeinen Formel I, in der m die Zahl 1 oder 2 darstellt:

Oxidation einer Verbindung der allgemeinen Formel

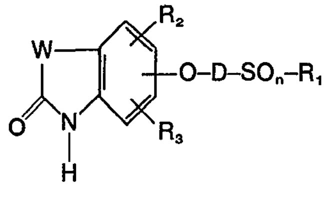

(IV),

in der

$R_1 - R_3$, D und W wie eingangs definiert sind und n die Zahl 0 oder 1 darstellt.

Die Oxidation wird vorzugsweise in einem Lösungsmittel, z. B. in Wasser, Wasser/Pyridin, Äthanol, Methanol, Aceton, Eisessig, Ameisensäure, verdünnter Schwefelsäure oder Trifluoressigsäure, je nach dem verwendeten Oxidationsmittel

zweckmässigerweise bei Temperaturen zwischen –80 und 100 °C durchgeführt.

Zur Herstellung von Verbindungen der allgemeinen Formel I, in der m die Zahl 1 darstellt, wird die Oxidation zweckmässigerweise mit einem Äquivalent des verwendeten Oxidationsmittels durchgeführt, z. B. mit Wasserstoffperoxid in Eisessig oder Ameisensäure bei 0 bis 20 °C oder in Aceton bei 0 bis 60 °C, mit einer Persäure wie Perameisensäure in Eisessig oder Trifluoressigsäure bei 0 bis 50 °C, mit Natriummetaperjodat in wässrigem Methanol oder Äthanol bei 15 bis 25 °C, mit N-Brom-succinimid in Äthanol, mit tert.-Butyl-hypochlorit in Methanol bei –80 bis –30 °C, mit Jodbenzoldichlorid in wässrigem Pyridin bei 0 bis 50 °C, mit Salpetersäure in Eisessig bei 0 bis 20 °C, mit Chromsäure in Eisessig oder in Aceton bei 0 bis 20 °C und mit Sulfurylchlorid in Methylenchlorid bei –70 °C, der hierbei erhaltene Thioäther-Chlor-Komplex wird zweckmässigerweise mit wässrigem Äthanol hydrolysiert.

Zur Herstellung von Verbindungen der allgemeinen Formel I, in der m die Zahl 2 darstellt, wird die Oxidation zweckmässigerweise mit einem bzw. mit zwei oder mehr Äquivalenten des verwendeten Oxidationsmittels durchgeführt, z. B. mit Wasserstoffperoxid in Eisessig oder in Ameisensäure bei 20 bis 100 °C oder in Aceton bei 0 bis 60 °C, mit einer Persäure wie Perameisensäure oder m-Chlorperbenzoesäure in Eisessig, Trifluoressigsäure oder Chloroform bei Temperaturen zwischen 0 und 50 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C. Bedeutet somit in einer Verbindung der obigen allgemeinen Formel IV n die Zahl 0, so wird die Umsetzung vorzugsweise mit zwei oder mehr Äquivalenten des betreffenden Oxidationsmittels und ganz entsprechend mit mindestens einem Äquivalent durchgeführt, falls n die Zahl 1 bedeutet.

Zur Herstellung einer Verbindung der allgemeinen Formel I, in der m die Zahl 0 darstellt:

Umsetzung einer Verbindung der allgemeinen Formel

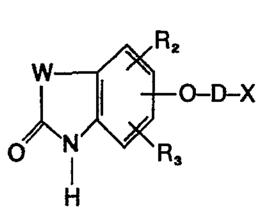 (V),

in der

$R_2$, $R_3$, D und W wie eingangs definiert sind, mit einer Verbindung der allgemeinen Formel

$$Y - R_1 \qquad (VI),$$

in der

$R_1$ wie eingangs definiert ist und

einer der Reste X oder Y in den Verbindungen der allgemeinen Formeln V und VI die Mercaptogruppe und der andere der Reste X oder Y eine nukleophil austauschbare Gruppe wie ein Halogenatom oder einen Sulfonsäureesterrest, z. B. ein Chlor-, Brom- oder Jodatom, eine p-Toluolsulfonyloxy- oder Methansulfonyloxygruppe, darstellt.

Die Umsetzung wird zweckmässigerweise in einem geeigneten Lösungsmittel wie Dioxan, Tetrahydrofuran, Chloroform oder Toluol, vorzugsweise jedoch in einem wasserfreien aprotischen Lösungsmittel wie Aceton, Dimethylformamid oder Dimethylsulfoxid, gegebenenfalls in Gegenwart einer Alkalibase wie Natriumkarbonat, Kaliumcarbonat oder Natriumhydroxid bei Temperaturen zwischen 0 °C und der Siedetemperatur des verwendeten Lösungsmittel, z. B. bei Temperaturen zwischen 0 und 100 °C, vorzugsweise jedoch bei Temperaturen zwischen 10 und 50 °C, durchgeführt. Die Umsetzung kann jedoch auch ohne Lösungsmittel durchgeführt werden.

d) Zur Herstellung eines Carbostyrils der allgemeinen Formel I, in der W die Vinylengruppe bedeutet:

Dehydrierung eines 3,4-Dihydro-carbostyrils der allgemeinen Formel

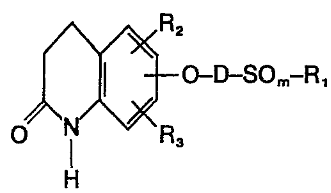 (VII),

in der

$R_1 - R_3$, D und m wie eingangs definiert sind.

Die Dehydrierung wird in Gegenwart eines Dehydrierungsmittels, z. B. einem Oxidationsmittel wie 2,3-Dichlor-5,6-di-cyano-benzochinon, Chloranil oder eines Edelmetallkatalysators wie Palladium/Kohle, vorzugsweise in einem inerten Lösungsmittel wie Dioxan oder Mesitylen bei erhöhten Temperaturen, z. B. bei Temperaturen zwischen 100 und 200 °C, vorzugsweise jedoch bei der Siedetemperatur des verwendeten Lösungsmittels, durchgeführt.

e) Zur Herstellung eines Carbostyrils der allgemeinen Formel I, in der W die Äthylengruppe und m die Zahl 0 oder 2 darstellen:

Hydrierung eines Carbostyrils der allgemeinen Formel

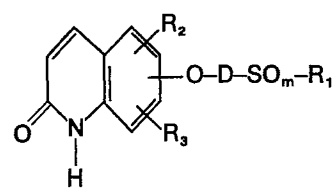 (VIII),

in der

$R_1 - R_3$, D und m wie eingangs definiert sind.

Die Hydrierung wird in einem geeigneten Lösungsmittel wie Äthanol, Essigsäureäthylester, Eisessig oder Dioxan mit katalytisch angeregtem Wasserstoff, z. B. mit Wasserstoff in Gegenwart eines Hydrierungskatalysators wie Palladium/Kohle, Platin, Raney-Nickel, Raney-Cobalt oder Dirhenium-haptasulfid, bei Temperaturen zwischen 0 und 50 °C, vorzugsweise jedoch bei Raumtemperatur, und bei einem Wasserstoffdruck von 1 bis 5 bar durchgeführt.

Die als Ausgangsstoffe verwendeten Verbindungen der allgemeinen Formeln II bis VIII sind teilweise literaturbekannt. Die übrigen erhält man nach an und für sich bekannten Verfahren. Beispielsweise erhält man ein 6-, 7- oder 8-Hydroxy-3,4-dihydro-carbostyril der allgemeinen Formel II durch Acylierung eines entsprechenden Anilinderivates mit einem entsprechenden β-Halogencarbonsäurederivat und anschliessende Cyclisierung nach Friedel-Crafts [siehe J. chem. Soc. 1955, 743–744, Chem. Pharm. Bull 1961, 970–975 und Ber. dtsch. Chem. Ges. 60, 858 (1927)] bzw. ein 5-Hydroxy-3,4-dihydrocarbostyril der allgemeinen Formel II durch Cyclisierung eines entsprechenden 2-(β-Cyanoäthyl)-cyclohexandion-1,3-Derivates und anschliessende Aromatisierung beispielsweise mit N-Brom-succinimid (siehe Chem. and Ind. 1970, 1435). Die Herstellung der entsprechenden Hydroxy-carbostyrile der allgemeinen Formel II ist literaturbekannt [siehe beispielsweise J. Amer. chem. Soc. 72, 346 (1950) und ibid 76, 2402 (1954) bzw. J. Org. Chem. 33, 1089 (1968) und ibid 36, 3493 (1971)]. Ferner wird die Herstellung von 5-Hydroxyoxindol in J. chem. 1961, 2723 beschrieben.

Die als Ausgangsstoffe verwendeten Verbindungen der allgemeinen Formeln IV, V, VII und VIII erhält man durch Alkylierung eines entsprechenden Hydroxyderivates und gegebenenfalls anschliessender Oxidation.

Wie bereits eingangs erwähnt, weisen die erfindungsgemäss hergestellten neuen Verbindungen der allgemeinen Formel I wertvolle pharmakologische Eigenschaften auf, neben einer positiv inotropen Wirkung besitzen sie antithrombotische Eigenschaften. Ausserdem stellen die Thioäther der allgemeinen Formel I wertvolle Zwischenprodukte zur Herstellung der Sulfoxide und Sulfone der allgemeinen Formel I dar.

Beispielsweise wurden die folgenden Verbindungen

A = 6-(4-Phenylmercapto-butoxy)-3,4-dihydrocarbostyril,
B = 6-(4-Phenylsulfinylbutoxy)-3,4-dihydrocarbostyril,
C = 6-(4-Phenylsulfonylbutoxy)-3,4-dihydrocarbostyril,
D = 6-[4-(2-Pyridylmercapto)-butoxy]-3,4-dihydrocarbostyril,
E = 6-[4-(2-Pyridylsulfinyl)-butoxy]-3,4-dihydrocarbostyril,
F = 6-[4-(2-Pyridylsulfonyl)-butoxy]-3,4-dihydrocarbostyril,
G = 6-(2-Phenylsulfinyl-äthoxy)-3,4-dihydrocarbostyril,
H 6-(4-Benzylsulfinyl-butoxy)-3,4-dihydrocarbostyril,
I = 6-[4-(4-Chlorphenylsulfinyl)-butoxy]-3,4-dihydrocarbostyril,
K = 6-(4-Cyclohexylsulfinyl-butoxy)-3,4-dihydrocarbostyril,
L = 6-[4-(2-Naphthylsulfinyl)-butoxy]-3,4-dihydrocarbostyril,
M = 6-[4-(2-Methoxyphenylsulfinyl)-butoxy]-3,4-dihydrocarbostyril,
N = 6-(4-Phenylsulfinyl-butoxy)-carbostyril,
O = 6-[4-(4-Hydroxy-3,5-di-tert.butyl-phenylsulfinyl)-butoxy]-carbostyril,
P = 6-[4-(3,4-Dichlorphenylsulfinyl)-butoxy]-carbostyril,
Q = 4-Methyl-6-(4-phenylsulfinyl-butoxy)-carbostyril,
R = 6-[4-(3,4-Dichlorphenylsulfonyl)-butoxy]-3,4-dihydrocarbostyril,
S = 6-[4-(2,5-Dichlorphenylsulfinyl)-butoxy]-3,4-dihydrocarbostyril,
T = 6-[4-(2-Pyridil-sulfonyl)-butoxy]-carbostyril,
U = 6-[4-(3,4-Dichlorphenylsulfinyl)-butoxy]-3,4-dihydrocarbostyril,

auf ihre biologischen Eigenschaften wie folgt untersucht:

1. Bestimmung der Thrombozytenaggregation nach Born und Cross [J. Physiol. 170, 397 (1964)]:

Die Thrombozytenaggregation wurde im plättchenreichen Plasma gesunder Versuchspersonen gemessen. Hierbei wurde der Verlauf der Abnahme der optischen Dichty nach Zugabe von Adenosindiphosphat (ADP) oder Collagen photometrisch gemessen und registriert. Aus dem Neigungswinkel der Dichtekurve wurde auf die Aggregationsgeschwindigkeit (Vmax) geschlossen. Der Punkt der Kurve, bei dem die grösste Lichtdurchlässigkeit vorlag, diente zur Berechnung der «optical density» (O.D.). Die $EC_{50}$-Angaben in den Tabellen beziehen sich auf die optical density.

Die Collagen-Dosen wurden möglichst gering gewählt, aber doch so, dass sich eine irreversible Aggregation ergab. Zur Aggregationsauslösung werden ca. 0,01 ml der Collagenlösung zu 1 ml plättchenreichem Plasma gegeben. (Handelsübliches Collagen der Fa. Hormonchemie, München). Die ADP-Dosen wurden so gewählt, dass sich nur die erste Phase der BORN-Kurve ergab. Die notwendige ADP-Menge lag bei ca. $1.10^{-6}$ Mol/l. Verwendet wurde handelsübliches ADP der Fa. Boehringer-Mannheim.

Es wurde die Substanz-Dosis graphisch bestimmt, die eine 50%ige Hemmung der Thrombozytenaggregation ($EC_{50}$) bewirkte:

| Verbindung | $EC_{50}$ $10^{-6}$ Mol/l Collagen | ADP |
|---|---|---|
| A | 50 | >100 |
| B | 4 | 20 |
| C | 5 | 15 |
| D | 45 | >100 |
| E | 6,5 | 25 |
| F | 20 | 20 |
| G | 3,5 | 17 |
| H | 2,5 | 14 |
| I | 4 | 10 |
| K | 4,5 | 12 |
| L | 1 | 10 |
| M | 4 | 22 |

| Verbindung | $EC_{50}$ $10^{-6}$ Mol/l Collagen | ADP |
|---|---|---|
| N | 0,6 | 3,0 |
| O | 0,2 | 0,5 |
| P | 0,2 | 1,8 |
| Q | 3,6 | 40 |
| R | 1,5 | 25 |
| S | 1,0 | 10 |
| T | 0,1 | 6 |
| U | 4 | 15 |

2. Bestimmung der positiv inotropen Wirkung:

Ratten wurden mit Äther narkotisiert und anschliessend durch Genickschlag getötet. Nach Eröffnen des Thorax wurde das Herz entnommen und beide Vorhöfe abgetrennt. Die Vorhöfe wurden in ein Organbad von 100 ml Inhalt überführt, das Tyrode von 30 °C enthält, die mit 95% $O_2$ und 5% $CO_2$ oxygeniert war. Die Vorhöfe schlugen spontan. Die Messung der Kontraktionskraft erfolgte isometrisch bei einer Belastung der Vorhöfe von 1 g.

Die zu testenden Substanzen wurden mit $1 \times 10^{-5}$ g/ml an jeweils 4 Vorhöfen geprüft. Die Änderungen der Kontraktionskraft wurden in % der Ausgangswerte ermittelt.

Die nachfolgende Tabelle enthält die gefundenen Werte:

| Substanz | Zunahme der Kontraktionskraft in % an der Ratte |
|---|---|
| B | 30 |
| C | 35 |
| I | 52 |
| L | 63 |
| M | 65 |
| Q | 58 |
| S | 83 |
| T | 32 |

3. Akute Toxizität:

Die akute Toxizität der zu untersuchenden Substanzen wurde orientierend an Gruppen von je 10 Mäusen nach oraler Gabe einer Dosis von 1000 mg/kg bestimmt (Beobachtungszeit: 14 Tage):

| Substanz | Akute Toxizität per os |
|---|---|
| A | $>$1000 mg/kg (0 von 10 Tieren gestorben) |
| B | $>$1000 mg/kg (0 von 10 Tieren gestorben) |
| C | $>$1000 mg/kg (0 von 10 Tieren gestorben) |
| D | $>$1000 mg/kg (0 von 10 Tieren gestorben) |
| E | $>$1000 mg/kg (0 von 10 Tieren gestorben) |
| F | $>$1000 mg/kg (0 von 10 Tieren gestorben) |
| G | $>$1000 mg/kg (0 von 10 Tieren gestorben) |
| H | $>$1000 mg/kg (0 von 10 Tieren gestorben) |
| I | $>$1000 mg/kg (0 von 10 Tieren gestorben) |
| K | $>$1000 mg/kg (0 von 10 Tieren gestorben) |
| L | $>$1000 mg/kg (0 von 10 Tieren gestorben) |
| M | $>$1000 mg/kg (0 von 10 Tieren gestorben) |
| O | $>$1000 mg/kg (0 von 10 Tieren gestorben) |
| P | $>$1000 mg/kg (0 von 10 Tieren gestorben) |
| Q | $>$1000 mg/kg (0 von 10 Tieren gestorben) |
| R | $>$1000 mg/kg (0 von 10 Tieren gestorben) |
| S | $>$1000 mg/kg (0 von 10 Tieren gestorben) |
| T | $>$1000 mg/kg (0 von 10 Tieren gestorben) |
| U | $>$1000 mg/kg (0 von 10 Tieren gestorben) |

Aufgrund ihrer pharmakologischen Eigenschaften eignen sich die neuen Verbindungen der allgemeinen Formel I zur Prophylaxe thrombo-embolischer Erkrankungen wie Coronarinfarkt, Cerebralinfarkt, sog. transient ischaemic attacks, Amaurosis fugax sowie zur Prophylaxe der Arteriosklerose, und lassen sich hierzu gegebenenfalls in Kombination mit anderen Wirksubstanzen in die üblichen pharmazeutischen Zubereitungsformen wie Dragées, Tabletten, Kapseln, Suppositorien oder Suspensionen einarbeiten. Die Einzeldosis beträgt hierbei 50 bis 100 mg 2–3 × täglich und die Tagesdosis somit 100–300 mg.

Die nachfolgenden Beispiele sollen die Erfindung näher erläutern:

Beispiel 1

6-[4-(2-Pyridylmercapto)-butoxy]-3,4-dihydrocarbostyril

14,4 g (0,13 Mol) 2-Mercaptopyridin und 17,9 g (0,13 Mol) Kaliumcarbonat werden in 360 ml über Molekularsieb getrocknetem Dimethylsulfoxid gerührt und mit 36 g (0,12 Mol) 6-(4-Brombutoxy)-3,4-dihydrocarbostyril (Schmelzpunkt: 142–147 °C, hergestellt aus 6-Hydroxy-carbostyril und 1,4-Dibrombutan) versetzt. Anschliessend rührt man 15 Stunden bei ca. 25 °C, giesst dann das Reaktionsgemisch in 3,6 l Wasser und rührt nochmals 30 Minuten. Das ausgefallene Produkt wird abgesaugt, gut mit Wasser gewaschen, getrocknet und unter Kohlebehandlung aus Xylol umkristallisiert.

Man erhält hellgelbe Kristalle vom Schmelzpunkt 123–124,5 °C.

Ausbeute: 32 g (81,2% der Theorie).

Beispiel 2
6-[4-(2-Pyridylsulfinyl)-butoxy]-3,4-dihydrocarbostyril

32,8 g (0,1 Mol) 6-[4-(2-Pyridylmercapto)-butoxy]-3,4-dihydrocarbostyril werden in 330 ml Eisessig gelöst und mit 10,2 g (0,105 Mol) 35%igem Wasserstoffperoxid versetzt. Man rührt 15 Stunden bei ca. 20 °C, destilliert dann bei 60 °C im Vakuum den Eisessig ab, wäscht den Rückstand mit Äther und kristallisiert das so erhaltene Rohprodukt 2 mal aus Xylol unter Kohlebehandlung um. Man erhält farblose Kristalle vom Schmelzpunkt 144,5–146 °C.

Ausbeute: 27,5 g (79,8% der Theorie).

Beispiel 3
6-[4-(2-Pyridylsulfonyl)-butoxy]-3,4-dihydrocarbostyril

5 g (0,015 Mol) 6-[4-(2-Pyridylmercapto)-butoxy]-3,4-dihydrocarbostyril werden in 50 ml Eisessig gelöst und mit 4,5 g (0,045 Mol) 35%igem Wasserstoffperoxid versetzt. Man rührt 40 Stunden bei ca. 25 °C, destilliert den Eisessig bei 60 °C im Vakuum ab, wäscht den festen Rückstand mit Äther und kristallisiert aus Xylol unter Kohlebehandlung um. Man erhält farblose Kristalle vom Schmelzpunkt 123,8–125 °C.

Ausbeute: 3,8 g (70,3% der Theorie).

Beispiel 4
6-(4-Phenylsulfinylbutoxy)-3,4-dihydrocarbostyril

32,6 g (0,2 Mol) 6-Hydroxy-3,4-dihydrocarbostyril [siehe F. Mayer et al. in Ber. dtsch. chem. Ges. 60, 858 (1927)] und 27,6 g (0,2 Mol) Kaliumcarbonat werden in 600 ml über Molekularsieb getrocknetem Dimethylsulfoxid 5 Minuten gerührt und dann mit 52,2 g 4-Phenylsulfinylbutylbromid (0,2 Mol) (hergestellt aus Thiophenol und 1,4-Dibrombutan und nachfolgender Oxidation mit Wasserstoffperoxid in Eisessig analog Beispiel 2; ölige Substanz, erstarrt beim Stehen im Kühlschrank) versetzt. Man lässt 15 Stunden bei 25 °C rühren und giesst dann das Reaktionsgemisch in 6 l Wasser. Anschliessend rührt man noch 30 Minuten, saugt das ausgefallene Produkt ab und wäscht gut mit Wasser. Nach dem Trocknen kristallisiert man unter Kohlebehandlung aus ca. 600 ml Xylol um. Man erhält weisse Kristalle vom Schmelzpunkt 144,5–145,5 °C.

Ausbeute: 49 g (71,3% der Theorie).

Beispiel 5
6-(4-Phenylmercapto-butoxy)-3,4-dihydrocarbostyril

Hergestellt analog Beispiel 4 aus 6-Hydroxy-3,4-dihydro-carbostyril und 4-(Phenylmercapto)-butylbromid ($Kp_{0,02}$:96–103 °C, hergestellt aus Thiophenol und 1,4-Dibrombutan).

Schmelzpunkt: 121,5–123 °C,

Ausbeute: 75,6% der Theorie.

Beispiel 6
6-(4-Phenylsulfonyl-butoxy)-3,4-dihydrocarbostyril

Hergestellt analog Beispiel 4 aus 6-Hydroxy-3,4-dihydrocarbostyril und 4-Phenyl-sulfonyl-butylbromid [hergestellt aus 4-(Phenylmercapto)-butylbromid durch Oxidation analog Beispiel 3].

Schmelzpunkt: 157,5–158 °C,

Ausbeute: 65,1% der Theorie.

Beispiel 7
6-[4-(4-Fluorphenylmercapto)-butoxy]-3,4-dihydrocarbostyril

Hergestellt analog Beispiel 1 aus 6-(4-Brombutoxy)-3,4-dihydrocarbostyril (Schmelzpunkt: 142–147 °C) und 4-Fluorthiophenol.

Schmelzpunkt: 139–140 °C,

Ausbeute: 93,1% der Theorie.

Beispiel 8
6-[4-(4-Fluorphenylsulfinyl)-butoxy]-3,4-dihydrocarbostyril

Hergestellt analog Beispiel 2 aus 6-[4-(4-Fluorphenylmercapto)-butoxy]-3,4-dihydrocarbostyril.

Schmelzpunkt: 184,5–186 °C,

Ausbeute: 88% der Theorie.

Beispiel 9
6-[4-(4-Methylphenyl-mercapto)-butoxy]-3,4-dihydrocarbostyril

Hergestellt analog Beispiel 1 aus 6-(4-Chlorbutoxy)-3,4-dihydrocarbostyril (Schmelzpunkt: 147–148 °C) und 4-Methylthiophenol.

Schmelzpunkt 120–121 °C,

Ausbeute: 91% der Theorie.

Beispiel 10
6-[4-(4-Methylphenyl-sulfinyl)-butoxy]-3,4-dihydrocarbostyril

Hergestellt analog Beispiel 2 aus 6-[4-(4-Methylphenylmercapto)-butoxy]-3,4-dihydrocarbostyril und Wasserstoffperoxid.

Schmelzpunkt: 149,5–150 °C,

Ausbeute: 97% der Theorie.

Beispiel 11
6-[4-(3-Methylphenylmercapto)-butoxy]-3,4-dihydrocarbostyril

Hergestellt analog Beispiel 1 aus 6-(4-Chlorbutoxy)-3,4-dihydrocarbostyril (Schmelzpunkt: 147–148 °C) und 3-Methylthiophenol.

Schmelzpunkt: 95–96 °C,

Ausbeute: 91% der Theorie.

Beispiel 12
6-[4-(3-Methylphenylsulfinyl)-butoxy]-3,4-dihydrocarbostyril

Hergestellt analog Beispiel 2 aus 6-[4-(3-Methylphenylmercapto)-butoxy]-3,4-dihydrocarbostyril und Wasserstoffperoxid.

Wachsartiges Harz,

Ausbeute: 95% der Theorie.

$R_f$-Wert: 0,48 (Dünnschichtchromatogramm an Kieselgel – Laufmittel: Benzol/Äthanol/konz. Ammoniak = 75/25/1).

Beispiel 13
6-[4-(4-Chlorphenylmercapto)-butoxy]-3,4-dihydrocarbostyril

Hergestellt analog Beispiel 1 aus 6-(4-Chlorbutoxy)-3,4-dihydrocarbostyril (Schmelzpunkt: 147–148 °C) und 4-Chlorthiophenol.
Schmelzpunkt: 144–146 °C,
Ausbeute: 88% der Theorie.

Beispiel 14
6-[4-(4-Chlorphenylsulfinyl)-butoxy]-3,4-dihydrocarbostyril

Hergestellt analog Beispiel 2 aus 6-[4-(4-Chlorphenylmercapto)-botoxy]-3,4-dihydrocarbostyril und Wasserstoffperoxid.
Schmelzpunkt: 148–149,5 °C,
Ausbeute: 70% der Theorie.

Beispiel 15
6-[4-(3,4-Dichlorphenylmercapto)-butoxy]-3,4-dihydrocarbostyril

Hergestellt analog Beispiel 1 aus 6-(4-Chlorbutoxy)-3,4-dihydrocarbostyril (Schmelzpunkt: 147–148 °C) und 3,4-Dichlorthiophenol.
Schmelzpunkt: 116,5–118 °C,
Ausbeute: 87% der Theorie.

Beispiel 16
6-[4-(3,4-Dichlorphenylsulfinyl)-butoxy]-3,4-dihydrocarbostyril

Hergestellt analog Beispiel 2 aus 6-[4-(3,4-Dichlorphenylmercapto)-butoxy]-3,4-dihydrocarbostyril und Wasserstoffperoxid.
Schmelzpunkt: 106,5–108 °C,
Ausbeute: 74% der Theorie.

Beispiel 17
6-[4-(2-Methoxyphenylmercapto)-butoxy]-3,4-dihydrocarbostyril

Hergestellt analog Beispiel 1 aus 6-(4-Brombutoxy)-3,4-dihydrocarbostyril (Schmelzpunkt: 142–147 °C) und 2-Methoxythiophenol.
Schmelzpunkt: 130,5–133 °C,
Ausbeute: 74% der Theorie.

Beispiel 18
6-[4-(2-Methoxyphenylsulfinyl)-butoxy]-3,4-dihydrocarbostyril

Hergestellt analog Beispiel 2 aus 6-[4-(2-Methoxyphenylmercapto)-butoxy]-3,4-dihydrocarbostyril und Wasserstoffperoxid.
Schmelzpunkt: 162–163 °C,
Ausbeute: 62% der Theorie.

Beispiel 19
6-[4-(3-Methoxyphenylmercapto)-butoxy]-3,4-dihydrocarbostyril

Hergestellt analog Beispiel 1 aus 6-(4-Brombutoxy)-3,4-dihydrocarbostyril (Schmelzpunkt: 142–147 °C) und 3-Methoxythiophenol.
Schmelzpunkt: 93,5–97 °C,
Ausbeute: 61% der Theorie.

Beispiel 20
6-[4-(3-Methoxyphenylsulfinyl)-butoxy]-3,4-dihydrocarbostyril

Hergestellt analog Beispiel 2 aus 6-[4-(3-Methoxyphenylmercapto)-butoxy]-3,4-dihydrocarbostyril und Wasserstoffperoxid.
Schmelzpunkt: 147–148 °C,
Ausbeute: 49% der Theorie.

Beispiel 21
6-[4-(4-Methoxyphenylmercapto)-butoxy]-3,4-dihydrocarbostyril

Hergestellt analog Beispiel 1 aus 6-(4-Brombutoxy)-3,4-dihydrocarbostyril (Schmelzpunkt: 142–147 °C) und 4-Methoxythiophenol.
Schmelzpunkt: 130,5–133 °C,
Ausbeute: 82% der Theorie.

Beispiel 22
6-[4-(4-Methoxyphenylsulfinyl)-butoxy]-3,4-dihydrocarbostyril

Hergestellt analog Beispiel 2 aus 6-[4-(4-Methoxyphenylmercapto)-butoxy]-3,4-dihydrocarbostyril und Wasserstoffperoxid.
Schmelzpunkt: 132–133 °C,
Ausbeute: 71% der Theorie.

Beispiel 23
6-[4-(3,4-Dimethoxyphenylmercapto)-butoxy]-3,4-dihydrocarbostyril

Hergestellt analog Beispiel 1 aus 3,4-Dimethoxythiophenol und 6-(4-Chlorbutoxy)-3,4-dihydrocarbostyril [hergestellt aus 6-Hydroxy-carbostyril (siehe F. Mayer et al. in Ber. dtsch. chem. Ges. 60, 858 (1927)) und 4-Chlorbutanol-benzolsulfonsäureester].
Schmelzpunkt: 117–119 °C,
Ausbeute: 73% der Theorie.

Beispiel 24
6-[4-(3,4-Dimethoxyphenylsulfinyl)-butoxy]-3,4-dihydrocarbostyril

Hergestellt analog Beispiel 2 aus 6-[4-(3,4-Dimethoxyphenylmercapto)-butoxy]-3,4-dihydrocarbostyril und Wasserstoffperoxid.
Schmelzpunkt: 145–147 °C,
Ausbeute: 79% der Theorie.

Beispiel 25
6-[4-(3,4-Dimethoxyphenylsulfonyl)-butoxy]-3,4-dihydrocarbostyril

Hergestellt analog Beispiel 3 aus 6-[4-(3,4-Dimethoxyphenylmercapto)-butoxy]-3,4-dihydrocarbostyril und Wasserstoffperoxid.
Schmelzpunkt: 158–160 °C,
Ausbeute: 62% der Theorie.

Beispiel 26
6-[4-(4-Biphenylylmercapto)-butoxy]-3,4-dihydrocarbostyril

Hergestellt analog Beispiel 1 aus 4-Phenylthiophenol und 6-(4-Brombutoxy)-3,4-dihydrocarbostyril.
Schmelzpunkt: 179,5–181 °C,
Ausbeute: 74% der Theorie.

Beispiel 27

6-[4-(4-Biphenylylsulfinyl)-butoxy]-3,4-dihydrocarbostyril

Hergestellt analog Beispiel 2 aus 6-[4-(4-Biphenylylmercapto)-butoxy]-3,4-dihydrocarbostyril und Wasserstoffperoxid.

Schmelzpunkt: 192–192,5 °C,
Ausbeute: 86% der Theorie.

Beispiel 28

6-[4-(2-Naphthylmercapto)-butoxy]-3,4-dihydrocarbostyril

Hergestellt analog Beispiel 1 aus 2-Naphthylmercaptan und 6-(4-Brombutoxy)-3,4-dihydrocarbostyril.

Schmelzpunkt: 108,5–109,5 °C,
Ausbeute: 48% der Theorie.

Beispiel 29

6-[4-(2-Naphthylsulfinyl)-butoxy]-3,4-dihydrocarbostyril

Hergestellt analog Beispiel 2 aus 6-[4-(2-Naphthylmercapto)-butoxy]-3,4-dihydrocarbostyril und Wasserstoffperoxid.

Schmelzpunkt: 147,5–148,5 °C,
Ausbeute: 57% der Theorie.

Beispiel 30

6-[5-(2-Pyridylsulfinyl)-pentoxy]-3,4-dihydrocarbostyril

Hergestellt analog Beispiel 2 aus 6-[5-(2-Pyridylmercapto)-pentoxy]-3,4-dihydrocarbostyril und Wasserstoffperoxid.

Schmelzpunkt: 116–118 °C,
Ausbeute: 69% der Theorie.

Beispiel 31

6-[5-(2-Pyridylsulfonyl)-pentoxy]-3,4-dihydrocarbostyril

Hergestellt analog Beispiel 3 aus 6-[5-(2-Pyridylmercapto)-pentoxy]-3,4-dihydrocarbostyril und Wasserstoffperoxid.

Schmelzpunkt 113,5–115,0 °C,
Ausbeute: 71% der Theorie.

Beispiel 32

7-(4-Phenylsulfonyl-butoxy)-carbostyril

Hergestellt analog Beispiel 3 aus 7-(4-Phenylmercapto-butoxy)-carbostyril und Wasserstoffperoxid.

Schmelzpunkt: 199–201 °C,
Ausbeute: 85% der Theorie.

Beispiel 33

6-(4-Cyclohexylmercaptobutoxy)-3,4-dihydrocarbostyril

Hergestellt analog Beispiel 1 aus Cyclohexylmercaptan und 6-(4-Chlorbutoxy)-3,4-dihydrocarbostyril (Schmelzpunkt: 147–148 °C).

Schmelzpunkt: 114–115 °C,
Ausbeute: 80% der Theorie.

Beispiel 34

6-(4-Cyclohexylsulfinyl-butoxy)-3,4-dihydrocarbostyril

Hergestellt analog Beispiel 2 aus 6-(4-Cyclohexylmercapto-butoxy)-3,4-dihydrocarbostyril und Wasserstoffperoxid.

Schmelzpunkt: 153–155,5 °C,
Ausbeute: 63% der Theorie.

Beispiel 35

6-(4-Benzylmercapto-butoxy)-3,4-dihydrocarbostyril

Hergestellt analog Beispiel 1 aus Benzylmercaptan und 6-(4-Chlorbutoxy)-3,4-dihydrocarbostyril.

Schmelzpunkt: 77,5–78,5 °C,
Ausbeute: 90% der Theorie.

Beispiel 36

6-(4-Benzylsulfinyl-butoxy)-3,4-dihydrocarbostyril

Hergestellt analog Beispiel 2 aus 6-(4-Benzylmercapto-butoxy)-3,4-dihydrocarbostyril und Wasserstoffperoxid.

Schmelzpunkt: 141,5–142 °C,
Ausbeute: 95% der Theorie.

Beispiel 37

6-[4-(2-Furylmethylmercapto)-butoxy]-3,4-dihydrocarbostyril

Hergestellt analog Beispiel 1 aus 2-Furfurylmercaptan und 6-(4-Brombutoxy)-3,4-dihydrocarbostyril (Schmelzpunkt: 142–147 °C).

Schmelzpunkt: 79–80 °C,
Ausbeute: 64% der Theorie.

Beispiel 38

6-[4-(2-Furylmethylsulfinyl)-butoxy]-3,4-dihydrocarbostyril

Hergestellt analog Beispiel 2 aus 6-[4-(2-Furylmethylmercapto)-butoxy]-3,4-dihydrocarbostyril und Wasserstoffperoxid.

Schmelzpunkt: 135–136 °C,
Ausbeute: 60% der Theorie.

Beispiel 39

6-[4-(2-Pyrimidyl-mercapto)-butoxy]-3,4-dihydrocarbostyril

Hergestellt analog Beispiel 1 aus 6-(4-Chlorbutoxy)-3,4-dihydrocarbostyril (Schmelzpunkt: 147–148 °C) und 2-Mercaptopyrimidin.

Schmelzpunkt: 154–156 °C,
Ausbeute: 79% der Theorie.

Beispiel 40

6-[4-(2-Pyrimidylsulfinyl)-butoxy]-3,4-dihydrocarbostyril

Hergestellt analog Beispiel 2 aus 6-[4-(2-Pyrimidylmercapto)-butoxy]-3,4-dihydrocarbostyril und Wasserstoffperoxid.

Schmelzpunkt: 154–156 °C,
Ausbeute: 36% der Theorie.

Beispiel 41

6-[4-(4-Pyridylmercapto)-butoxy]-3,4-dihydrocarbostyril

3,0 g 6-(4-Brombutoxy)-3,4-dihydrocarbostyril werden zu einer Lösung von 1,3 g 4-Mercaptopyridin mit 2,3 g 30%iger Natriummethyllatlösung in

15 ml Methanol hinzugefügt und bei Zimmertemperatur 14 Stunden gerührt. Dann verdünnt man mit 20 ml Wasser und kristallisiert den ausgefallenen Niederschlag aus Äthanol um.
Schmelzpunkt: 128–129 °C,
Ausbeute: 1,6 g (49% der Theorie).

Beispiel 42
6-[4-(2-Benzimidazolylmercapto)-butoxy]-3,4-dihydrocarbostyril
Hergestellt analog Beispiel 1 aus 6-(4-Chlorbutoxy)-3,4-dihydrocarbostyril (Schmelzpunkt: 147–148 °C) und 2-Mercaptobenzimidazol.
Schmelzpunkt: 100–103 °C,
Ausbeute: 45% der Theorie.

Beispiel 43
6-[4-(2-Benzimidazolylsulfinyl)-butoxy]-3,4-dihydrocarbostyril
Hergestellt analog Beispiel 2 aus 6-[4-(2-Benzimidazolylmercapto)-butoxy]-3,4-dihydrocarbostyril und Wasserstoffperoxid.
Schmelzpunkt: 180–182 °C,
Ausbeute: 36% der Theorie.

Beispiel 44
6-[4-(2-Benzthiazolyl-mercapto)-butoxy]-3,4-dihydrocarbostyril
Hergestellt analog Beispiel 1 aus 6-(4-Chlorbutoxy)-3,4-dihydrocarbostyril (Schmelzpunkt: 147–148 °C) und 2-Mercaptobenzthiazol.
Schmelzpunkt: 157–158 °C,
Ausbeute: 70% der Theorie.

Beispiel 45
6-[4-(2-Benzthiazolylsulfinyl)-butoxy]-3,4-dihydrocarbostyril
Hergestellt analog Beispiel 2 aus 6-[4-(2-Benzthiazolylmercapto)-butoxy]-3,4-dihydrocarbostyril und Wasserstoffperoxid.
Schmelzpunkt: 183–184 °C,
Ausbeute: 69% der Theorie.

Beispiel 46
6-(2-Phenylmercapto-äthoxy)-3,4-dihydrocarbostyril
Hergestellt analog Beispiel 1 aus Thiophenol und 6-(2-Chloräthoxy)-3,4-dihydrocarbostyril (Schmelzpunkt: 152,5–153,5 °C).
Schmelzpunkt: 132–133,5 °C,
Ausbeute: 91% der Theorie.

Beispiel 47
6-(2-Phenylsulfinyl-äthoxy)-3,4-dihydrocarbostyril
Hergestellt analog Beispiel 2 aus 6-(2-Phenylmercapto-äthoxy)-3,4-dihydrocarbostyril und Wasserstoffperoxid.
Schmelzpunkt: 171–172 °C,
Ausbeute: 84% der Theorie.

Beispiel 48
6-(2-Phenylsulfonyl-äthoxy)-3,4-dihydrocarbostyril
Hergestellt analog Beispiel 3 aus 6-(2-Phenylmercapto-äthoxy)-3,4-dihydrocarbostyril und Wasserstoffperoxid.
Schmelzpunkt: 185–186 °C,
Ausbeute: 94% der Theorie.

Beispiel 49
6-(3-Phenylmercapto-propoxy)-3,4-dihydrocarbostyril
Hergestellt analog Beispiel 1 aus 6-(3-Brompropoxy)-3,4-dihydrocarbostyril (Schmelzpunkt: 111–118 °C) und Thiophenol.
Schmelzpunkt: 111–112 °C,
Ausbeute: 77% der Theorie.

Beispiel 50
6-(3-Phenylsulfinyl-propoxy)-3,4-dihydrocarbostyril
Hergestellt analog Beispiel 2 aus 6-[3-(Phenylmercapto)-propoxy]-3,4-dihydrocarbostyril und Wasserstoffperoxid.
Schmelzpunkt: 131,5–133,5 °C,
Ausbeute: 67% der Theorie.

Beispiel 51
6-(6-Phenylmercapto-hexoxy)-3,4-dihydrocarbostyril
Hergestellt analog Beispiel 1 aus 6-(6-Bromhexoxy)-3,4-dihydrocarbostyril (Schmelzpunkt: 107,5–108 °C) und Thiophenol.
Schmelzpunkt: 112,5–113 °C,
Ausbeute: 34% der Theorie.

Beispiel 52
6-(6-Phenylsulfinyl-hexoxy)-3,4-dihydrocarbostyril
Hergestellt analog Beispiel 2 aus 6-(6-Phenylmercapto-hexoxy)-3,4-dihydrocarbostyril und Wasserstoffperoxid.
Schmelzpunkt: 119,5–121,5 °C,
Ausbeute: 35% der Theorie.

Beispiel 53
7-(4-Phenylmercaptobutoxy)-3,4-dihydrocarbostyril
Hergestellt analog Beispiel 4 aus 7-Hydroxy-3,4-dihydrocarbostyril (N. Shigematsu et al. in Chem. Pharm. Bull 1961, 970–975) und 4-Phenylmercapto-butylbromid ($Kp_{0,02}$:96–103 °C).
Schmelzpunkt: 121–123 °C,
Ausbeute: 72% der Theorie.

Beispiel 54
7-(4-Phenylsulfinyl-butoxy)-3,4-dihydrocarbostyril
Hergestellt analog Beispiel 4 aus 7-Hydroxy-3,4-dihydrocarbostyril (N. Shigematsu et al. in Chem. Pharm. Bull. 1961, 970–975) und 4-Phenylsulfinylbutylbromid.
Schmelzpunkt: 134–136 °C,
Ausbeute: 80% der Theorie.

Beispiel 55
7-(4-Phenylsulfonyl-butoxy)-3,4-dihydrocarbostyril
Hergestellt analog Beispiel 4 aus 7-Hydroxy-3,4-dihydrocarbostyril (N. Shigematsu et al. in Chem.

Pharm. Bull. 1961, 970–975) und 4-Phenylsulfonylbutylbromid.

Schmelzpunkt: 178,5–179,5 °C,

Ausbeute: 74% der Theorie.

Beispiel 56

8-(4-Phenylmercaptobutoxy)-3,4-dihydrocarbostyril

Hergestellt analog Beispiel 4 aus 8-Hydroxy-3,4-dihydrocarbostyril (J.D. Loudon et al. in J. Chem. Soc. 1955, 743–744) und 4-Phenylmercapto-butylbromid ($Kp_{0,02}$:96–103 °C).

Schmelzpunkt: 101–102 °C,

Ausbeute: 75% der Theorie.

Beispiel 57

8-(4-Phenylsulfinyl-butoxy)-3,4-dihydrocarbostyril

Hergestellt analog Beispiel 4 aus 8-Hydroxy-3,4-dihydrocarbostyril (J.D. Loudon et al. in J. Chem. Soc. 1955, 743–744) und Phenylsulfinylbutylbromid.

Farbloses Harz,

Ausbeute: 60% der Theorie.

$R_f$-Wert: 0,60 (Dünnschichtchromatogramm an Kieselgel – Laufmittel: Benzol/Äthanol/konz. Ammoniak = 75/25/1).

Beispiel 58

8-(4-Phenylsulfonyl-butoxy)-3,4-dihydrocarbostyril

Hergestellt analog Beispiel 3 aus 8-(4-Phenylmercapto-butoxy)-3,4-dihydrocarbostyril und Wasserstoffperoxid.

Schmelzpunkt: 114,5–115 °C,

Ausbeute: 60% der Theorie.

Beispiel 59

6-[4-(2-Benzthiazolyl-sulfonyl)-butoxy]-3,4-dihydrocarbostyril

Hergestellt analog Beispiel 2 aus 6-[4-(2-Benzthiazolyl-mercapto)-butoxy]-3,4-dihydrocarbostyril und Wasserstoffperoxid.

Schmelzpunkt: 146–149 °C,

Ausbeute: 61% der Theorie.

Beispiel 60

6-[4-(2-Chinolylmercapto)-butoxy]-3,4-dihydrocarbostyril

Hergestellt analog Beispiel 1 aus 6-(4-Brombutoxy)-3,4-dihydrocarbostyril (Schmelzpunkt: 142–147 °C) und 2-Mercaptochinolin.

Schmelzpunkt: 115 °C,

Ausbeute: 64% der Theorie.

Beispiel 61

6-[4-(2-Chinazolin-4-on-yl-mercapto)-butoxy]-3,4-dihydrocarbostyril

Hergestellt analog Beispiel 1 aus 6-(4-Brombutoxy)-3,4-dihydrocarbostyril (Schmelzpunkt: 142–147 °C) und 2-Mercaptochinazolin-4-on.

Schmelzpunkt: 184,5–188 °C,

Ausbeute: 63% der Theorie.

Beispiel 62

6-(4-Triphenylmethylmercapto-butoxy)-3,4-dihydrocarbostyril

Hergestellt analog Beispiel 1 aus 6-(4-Brombutoxy)-3,4-dihydrocarbostyril (Schmelzpunkt: 142–147 °C) und Triphenylmethylmercaptan.

Schmelzpunkt: 169–170 °C,

Ausbeute: 89% der Theorie.

Beispiel 63

6-[2-(2-Naphthylmercapto)-äthoxy]-3,4-dihydrocarbostyril

Hergestellt analog Beispiel 1 aus 6-(2-Chloräthoxy)-3,4-dihydrocarbostyril (Schmelzpunkt: 147–148 °C) und 2-Naphthylmercaptan.

Schmelzpunkt: 147,5–147,8 °C,

Ausbeute: 77% der Theorie.

Beispiel 64

6-[2-(2-Naphthylsulfinyl)-äthoxy]-3,4-dihydrocarbostyril

Hergestellt analog Beispiel 2 aus 6-[2-(2-Naphthylmercapto)-äthoxy]-3,4-dihydrocarbostyril und Wasserstoffperoxid.

Schmelzpunkt 186,5–187,5 °C,

Ausbeute: 88% der Theorie.

Beispiel 65

6-[2-(4-Biphenylylmercapto)-äthoxy]-3,4-dihydrocarbostyril

Hergestellt analog Beispiel 1 aus 6-(2-Chloräthoxy)-3,4-dihydrocarbostyril (Schmelzpunkt: 152,5–153,5 °C) und 4-Mercaptobiphenyl.

Schmelzpunkt: 192–194 °C,

Ausbeute: 92% der Theorie.

Beispiel 66

6-[2-(4-Biphenylyl-sulfinyl)-äthoxy]-3,4-dihydrocarbostyril

Hergestellt analog Beispiel 2 aus 6-[2-(4-Biphenylylmercapto)-äthoxy]-3,4-dihydrocarbostyril und Wasserstoffperoxid.

Schmelzpunkt: 195–196 °C,

Ausbeute: 66% der Theorie.

Beispiel 67

6-[3-(2-Pyridylmercapto)-propoxy]-3,4-dihydrocarbostyril

Hergestellt analog Beispiel 1 aus 6-(3-Brompropoxy)-3,4-dihydrocarbostyril und 2-Mercaptopyridin.

Schmelzpunkt: 108–108,5 °C,

Ausbeute: 42% der Theorie.

Beispiel 68

6-(4-Phenylsulfinyl-butoxy)-3,4-dihydrocarbostyril

1,6 g 6-(4-Phenylmercapto-butoxy)-3,4-dihydrocarbostyril werden in 50 ml Methanol gelöst und mit 0,9 g N-Bromsuccinimid versetzt. Man rührt bei Zimmertemperatur während 15 Stunden, verdünnt mit 500 ml 80 °C heissem Wasser und dekantiert von dem zunächst öligen Rückstand ab, der allmählich durchkristallisiert. Nach Umkristallisieren aus Xylol erhält man weisse Kristalle vom Schmelzpunkt 144–145 °C.

Ausbeute: 1,2 g (66% der Theorie).

Beispiel 69

6-(4-Phenylsulfinyl-butoxy)-3,4-dihydrocarbostyril

3,3 g 6-(4-Phenylmercapto-butoxy)-3,4-dihydrocarbostyril werden in 50 ml Methylenchlorid gelöst, auf -70 °C abgekühlt und dann mit einer Lösung von 1,5 g Sulfurylchlorid in 5 ml Methylenchlorid tropfenweise versetzt. Nach 15 Stunden wird mit 20 ml 95%igem Äthanol versetzt und durch Entfernen des Kühlbades auf Raumtemperatur erwärmt. Man neutralisiert mit wässriger Sodalösung, trocknet die Methylenchloridphase mit Natriumsulfat, dampft das Lösungsmittel ab und kristallisiert den Rückstand aus Toluol um.

Schmelzpunkt: 143–145 °C,
Ausbeute: 2,8 g (81% der Theorie).

Beispiel 70

5-(4-Phenylmercapto-butoxy)-3,4-dihydrocarbostyril

Hergestellt analog Beispiel 4 aus 5-Hydroxy-3,4-dihydrocarbostyril und 4-Phenylmercaptobutylbromid.

Schmelzpunkt: 155–157 °C,
Ausbeute: 64% der Theorie.

Beispiel 71

5-(4-Phenylsulfinyl-butoxy)-3,4-dihydrocarbostyril

Hergestellt analog Beispiel 4 aus 5-Hydroxy-3,4-dihydrocarbostyril und 4-Phenylsulfinylbutylbromid.

Schmelzpunkt: 136–138 °C,
Ausbeute: 64% der Theorie.

Beispiel 72

5-(4-Phenylsulfonyl-butoxy)-3,4-dihydrocarbostyril

Hergestellt analog Beispiel 4 aus 5-Hydroxy-3,4-dihydrocarbostyril und 4-Phenylsulfonylbutylbromid.

Schmelzpunkt: 187–189 °C,
Ausbeute: 73% der Theorie.

Beispiel 73

6-[4-(2-Naphthylsulfonyl)-butoxy]-3,4-dihydrocarbostyril

Hergestellt analog Beispiel 3 aus 6-[4-(2-Naphthylmercapto)-butoxy]-3,4-dihydrocarbostyril und Wasserstoffperoxid.

Schmelzpunkt: 173–175 °C,
Ausbeute: 95% der Theorie.

Beispiel 74

6-[4-(4-Biphenylylsulfonyl)-butoxy]-3,4-dihydrocarbostyril

Hergestellt analog Beispiel 3 aus 6-[4-(4-Biphenylylmercapto)-butoxy]-3,4-dihydrocarbostyril und Wasserstoffperoxid.

Schmelzpunkt: 232–234,5 °C,
Ausbeute: 83% der Theorie.

Beispiel 75

6-(4-Phenylsulfonyl-butoxy)-3,4-dihydrocarbostyril

Hergestellt analog Beispiel 3 aus 6-(4-Phenylsulfinyl-butoxy)-3,4-dihydrocarbostyril und Wasserstoffperoxid.

Schmelzpunkt: 157–158 °C,
Ausbeute: 88% der Theorie

Beispiel 76

6-[4-(2-Pyridylsulfonyl)-butoxy]-3,4-dihydrocarbostyril

Hergestellt analog Beispiel 3 aus 6-[4-(2-Pyridylsulfinyl)-butoxy]-3,4-dihydrocarbostyril und Wasserstoffperoxid.

Schmelzpunkt: 123,8–125 °C,
Ausbeute: 76% der Theorie.

Beispiel 77

5-(4-Phenylsulfonyl-butoxy)-carbostyril

1,8 g 5-(4-Phenylsulfonyl-butoxy)-3,4-dihydrocarbostyril werden in 45 ml Dioxan gelöst, mit 1,87 g 2,3-Dichlor-5,6-dicyanobenzochinon versetzt und im Ölbad 2,5 Stunden lang zum Sieden erhitzt. Man filtriert heiss und wäscht den unlöslichen Rückstand, der sich während der Reaktion gebildet hat, mit heissem Dioxan aus. Die vereinigten Filtrate werden mit 100 ml Chloroform verdünnt und mehrmals mit insgesamt 150 ml 2N-Natronlauge ausgeschüttelt. Nach dem Trocknen der Chloroformextrakte mit Natriumsulfat und Filtrieren in Gegenwart von Aktivkohle wird eingeengt und mit Essigester versetzt, bis sich weisse Kristalle abscheiden.

Schmelzpunkt: 182–183 °C,
Ausbeute: 850 mg (47% der Theorie).

Beispiel 78

5-(4-Phenylmercapto-butoxy)-carbostyril

Hergestellt analog Beispiel 77 aus 5-(4-Phenylmercapto-butoxy)-3,4-dihydrocarbostyril und 2,3-Dichlor-5,6-dicyano-benzochinon.

Schmelzpunkt: 185–187 °C,
Ausbeute: 40% der Theorie.

Beispiel 79

5-(4-Phenylsulfinyl-butoxy)-carbostyril

0,56 g 5-(4-Phenylmercapto-butoxy)-carbostyril werden mit 0,165 ml 30%igem Wasserstoffperoxid, gelöst in 8 ml Eisessig, versetzt, mit Wasser verdünnt und zweimal mit Chloroform extrahiert. Der Chloroformextrakt wird mit verdünnter Sodalösung, mit Wasser gewaschen, über Magnesiumsulfat getrocknet und eingeengt. Nach Versetzen mit Essigester erhält man farblose Kristalle vom Schmelzpunkt 155–157 °C.

Ausbeute: 389 mg (65% der Theorie).

Beispiel 80

6-(4-Phenylmercapto-butoxy)-carbostyril

Hergestellt analog Beispiel 77 aus 6-(4-Phenylmercapto-butoxy)-3,4-dihydrocarbostyril und 2,3-Dichlor-4,5-dicyano-benzochinon.

Schmelzpunkt: 162–164 °C,
Ausbeute: 35% der Theorie.

Beispiel 81

6-(4-Phenylsulfinyl-butoxy)-carbostyril

Hergestellt analog Beispiel 77 aus 6-(4-Phenylsulfinyl-butoxy)-3,4-dihydrocarbostyril und 2,3-Dichlor-5,6-dicyano-benzochinon. Zur Reinigung wird an einer Kieselgelsäule mit einem Gemisch von Benzol/Äthanol/konz. Ammoniak = 75/25/3 chromatographiert.

Schmelzpunkt: 181–182,5 °C,
Ausbeute: 48% der Theorie.

Aus den ersten Fraktionen der Säulenchromatographie isoliert man in einer Ausbeute von 15% der Theorie 6-(4-Phenylmercapto-butoxy)-carbostyril vom Schmelzpunkt 162–164 °C.

Beispiel 82

6-(4-Phenylsulfonyl-butoxy)-carbostyril

Hergestellt analog Beispiel 77 aus 6-(4-Phenylsulfonyl-butoxy)-3,4-dihydrocarbostyril und 2,3-Dichlor-5,6-dicyano-benzochinon. Die Reinigung erfolgt durch Säulenchromatographie an Kieselgel (Korngrösse: 0,2–0,5 mm) mit Chloroform/Methanol/Essigsäureäthylester = 4/1/1.

Schmelzpunkt: 212–213 °C,
Ausbeute: 54% der Theorie.

Beispiel 83

5-(4-Phenylmercapto-butoxy)-carbostyril

Eine Mischung aus 32,3 g 5-Hydroxycarbostyril, 30 g Kaliumcarbonat und 650 ml über Molekularsieb getrocknetem Dimethylsulfoxid wird mit 49 g 4-Phenylmercaptobutylbromid versetzt. Man rührt 20 Stunden bei 25 °C, verdünnt mit 3 Liter Wasser und saugt das auskristallisierte Reaktionsprodukt ab.

Schmelzpunkt 185–187 °C (aus Toluol),
Ausbeute: 45,0 g (70% der Theorie).

Beispiel 84

5-(4-Phenylsulfonyl-butoxy)-carbostyril

Hergestellt analog Beispiel 79 aus 5-(4-Phenylmercapto-butoxy)-carbostyril und 4 Mol Wasserstoffperoxid bei einer Temperatur von 60 °C und einer Reaktionszeit von 14 Stunden.

Schmelzpunkt: 182–183 °C,
Ausbeute: 73% der Theorie.

Beispiel 85

6-(4-Phenylmercapto-butoxy)-carbostyril

Hergestellt analog Beispiel 83 aus 6-Hydroxycarbostyril und 4-Phenylmercaptobutylbromid.

Schmelzpunkt: 161–163 °C,
Ausbeute: 78% der Theorie.

Beispiel 86

6-(4-Phenylsulfinyl-butoxy)-carbostyril

Hergestellt analog Beispiel 79 aus 6-(4-Phenylmercapto-butoxy)-carbostyril und Wasserstoffperoxid.

Schmelzpunkt: 181–182 °C,
Ausbeute: 68% der Theorie.

Beispiel 87

6-(4-Phenylsulfinyl-butoxy)-carbostyril

Hergestellt analog Beispiel 83 aus 6-Hydroxycarbostyril und 4-Phenylsulfinylbutylbromid.

Schmelzpunkt: 181–182 °C,
Ausbeute: 71% der Theorie.

Beispiel 88

7-(4-Phenylsulfinyl-butoxy)-carbostyril

Hergestellt analog Beispiel 77 aus 7-(4-Phenylsulfinyl-butoxy)-3,4-dihydrocarbostyril.

Schmelzpunkt: 193–194 °C,
Ausbeute: 51% der Theorie.

Beispiel 89

8-(4-Phenylmercapto-butoxy)-carbostyril

Hergestellt analog Beispiel 77 aus 8-(4-Phenylmercapto-butoxy)-3,4-dihydrocarbostyril und 2,3-Dichlor-5,6-dicyano-benzochinon.

Schmelzpunkt: 119–120 °C,
Ausbeute: 40% der Theorie.

Beispiel 90

8-(4-Phenylsulfinyl-butoxy)-carbostyril

Hergestellt analog Beispiel 79 aus 8-(4-Phenylmercapto-butoxy)-carbostyril durch Oxidation mit Wasserstoffperoxid.

Schmelzpunkt: 125,5–126,5 °C,
Ausbeute: 60% der Theorie.

Beispiel 91

5-(4-Phenylsulfonyl-butoxy)-carbostyril

100 mg 5-(4-Phenylsulfonyl-butoxy)-3,4-dihydrocarbostyril werden mit 30 mg Palladium/Kohle und 1 ml Mesitylen zum Sieden am Rückfluss erhitzt. Nach 3,5 Stunden werden weitere 30 mg Palladium/Kohle zugefügt und weitere 9 Stunden erhitzt. Danach wird heiss filtriert, das Filtrat eingedampft und der Rückstand an einer Kieselgelsäule mit einem Gemisch von Benzol/Äthanol/konz. Ammoniak = 75/25/3 chromatographiert. Man erhält neben unumgesetztem Ausgangsmaterial 9 mg 5-(4-Phenylsulfonyl-butoxy)-carbostyril vom Schmelzpunkt 182–183 °C.

Beispiel 92

6-(4-Amino-iminomethylmercapto-butoxy)-3,4-dihydrocarbostyril

18 g 6-(4-Brombutoxy)-3,4-dihydrocarbostyril werden zusammen mit 5 g Thioharnstoff in 250 ml Wasser so lange zum Sieden erhitzt bis alles in Lösung gegangen ist (ca. 4 Stunden). Nach dem Abkühlen extrahiert man die Verunreinigungen mit Chloroform und stellt mit Ammoniak alkalisch. Die ausgeschiedenen Kristalle werden abgesaugt und getrocknet.

Schmelzpunkt: 140–141,8 °C,
Schmelzpunkt des Hydrochlorids: 208–211 °C,
Ausbeute: 90% der Theorie.

Beispiel 93

6-(4-Phenylsulfonyl)-butoxy-3,4-dihydrocarbostyril

107 mg 6-(4-Phenylsulfonyl)-butoxy-carbostyril werden in 15 ml Methanol suspendiert, mit 40 mg Palladium/Kohle versetzt und bei 50 °C und einem Wasserstoffdruck von 2,5 bar 14 Stunden lang hydriert. Nach Abfiltrieren des Katalysators und Einengen der klaren Lösung erhält man farblose Kristalle.

Schmelzpunkt: 153–154 °C,
Ausbeute: 89 mg (83% der Theorie).

Beispiel 94

6-(4-Benzylmercapto-butoxy)-3,4-dihydrocarbostyril

2,5 g 6-(4-Mercaptobutoxy)-3,4-dihydrocarbostyril, gelöst in 25 ml Dimethylsulfoxid, werden unter Rühren mit 1,4 g Kaliumcarbonat und anschliessend mit 1,3 ml Benzylchlorid versetzt. Nach 15 Stunden Rühren bei Raumtemperatur, verdünnt man mit 200 ml Wasser, trennt die ausgeschiedene ölige Substanz ab und kristallisiert aus Essigester um.

Schmelzpunkt: 76–78 °C,
Ausbeute: 2,8 g (82% der Theorie).

Beispiel 95

6-(5-Phenylmercaptopentoxy)-3,4-dihydrocarbostyril

Hergestellt analog Beispiel 1 aus 6-(5-Brompentoxy)-3,4-dihydrocarbostyril und Thiophenol.

Schmelzpunkt: 117–119 °C,
Ausbeute: 71% der Theorie.

Beispiel 96

6-(5-Phenylsulfinyl-pentoxy)-3,4-dihydrocarbostyril

Hergestellt analog Beispiel 2 aus 6-(5-Phenylmercapto-pentoxy)-3,4-dihydrocarbostyril und Wasserstoffperoxid.

Schmelzpunkt: 104–109,5 °C,
Ausbeute: 66% der Theorie.

Beispiel 97

6-(5-Phenylsulfinyl-pentoxy)-3,4-dihydrocarbostyril

Hergestellt analog Beispiel 3 aus 6-(5-Phenylmercapto-pentoxy)-3,4-dihydrocarbostyril und Wasserstoffperoxid.

Schmelzpunkt: 136,5–137,8 °C,
Ausbeute: 62% der Theorie.

Beispiel 98

6-[5-(2-Pyridyl-mercapto)-pentoxy]-3,4-dihydrocarbostyril

Hergestellt analog Beispiel 1 aus 6-(5-Brompentoxy)-3,4-dihydrocarbostyril und 2-Mercaptopyridin.

Schmelzpunkt: 113–114,8 °C,
Ausbeute: 76% der Theorie.

Beispiel 99

6-[4-(4-Hydroxyphenylmercapto)-butoxy]-3,4-dihydrocarbostyril

Hergestellt analog Beispiel 1 aus 6-(4-Brombutoxy)-3,4-dihydrocarbostyril und 4-Hydroxythiophenol.

Schmelzpunkt: 191,5–193,0 °C,
Ausbeute: 83% der Theorie.

Beispiel 100

6-[4-(4-Hydroxyphenylsulfinyl)-butoxy]-3,4-dihydrocarbostyril

Hergestellt analog Beispiel 2 aus 6-[4-(4-Hydroxyphenylmercapto)-butoxy]-3,4-dihydrocarbostyril und Wasserstoffperoxid.

Schmelzpunkt: 206–207,8 °C,
Ausbeute: 84% der Theorie.

Beispiel 101

6-[4-(4-Hydroxyphenylsulfonyl)-butoxy]-3,4-dihydrocarbostyril

Hergestellt analog Beispiel 3 aus 6-[4-(4-Hydroxyphenylmercapto)-butoxy]-3,4-dihydrocarbostyril und Wasserstoffperoxid.

Schmelzpunkt: 219–219,5 °C,
Ausbeute: 78% der Theorie.

Beispiel 102

6-[4-(4-Acetaminophenylmercapto)-butoxy]-3,4-dihydrocarbostyril

Hergestellt analog Beispiel 1 aus 6-(4-Brombutoxy)-3,4-dihydrocarbostyril und 4-Acetaminothiophenol.

Schmelzpunkt: 162,5–163,0 °C,
Ausbeute: 65% der Theorie.

Beispiel 103

6-[4-(4-Acetaminophenylsulfinyl)-butoxy]-3,4-dihydrocarbostyril

Hergestellt analog Beispiel 2 aus 6-[4-(4-Acetaminophenylmercapto)-butoxy]-3,4-dihydrocarbostyril und Wasserstoffperoxid.

Schmelzpunkt: 202–203 °C,
Ausbeute: 55% der Theorie.

Beispiel 104

6-[4-(4-Acetaminophenylsulfonyl)-butoxy]-3,4-dihydrocarbostyril

Hergestellt analog Beispiel 3 aus 6-[4-(4-Acetaminophenylmercapto)-butoxy]-3,4-dihydrocarbostyril und Wasserstoffperoxid.

Schmelzpunkt: 143,5–147 °C,
Ausbeute: 88% der Theorie.

Beispiel 105

6-[4-(4,5-Di-p-chlorphenyl-oxazol-2-yl-mercapto)-butoxy]-3,4-dihydrocarbostyril

Hergestellt analog Beispiel 1 aus 6-(4-Brombutoxy)-3,4-dihydrocarbostyril und 2-Mercapto-4,5-di-p-chlorphenyl-oxazol.

Schmelzpunkt: 110–115 °C,
Ausbeute: 70% der Theorie.

Beispiel 106

6-[4-(2-Pyridylsulfinyl)-butoxy]-carbostyril

Hergestellt analog Beispiel 77 aus 6-[4-(2-Pyridylsulfinyl)-butoxy]-3,4-dihydrocarbostyril und 2,3-Dichlor-5,6-dicyano-benzochinon.

Schmelzpunkt: 152–154 °C,
Ausbeute: 48% der Theorie.

Beispiel 107

6-(4-Phenylmercapto-butoxy)-3,4-dihydrocarbostyril

Hergestellt analog Beispiel 93 aus 6-(4-Phenylmercapto-butoxy)-carbostyril durch katalytische Hydrierung mit Rhenium-VII-sulfid als Katalysator.

Schmelzpunkt: 121–122 °C,
Ausbeute: 67% der Theorie.

Beispiel 108

8-(4-Phenylsulfonyl-butoxy)-carbostyril

Hergestellt analog Beispiel 77 aus 8-(4-Phenylsulfonyl-butoxy)-3,4-dihydrocarbostyril und 2,3-Dichlor-5,6-dicyano-benzochinon.

Schmelzpunkt: 146–147 °C,

Ausbeute: 41% der Theorie.

Beispiel 109

7-(4-Phenylmercapto-butoxy)-carbostyril

Hergestellt analog Beispiel 77 aus 7-(4-Phenylmercaptobutoxy)-3,4-dihydrocarbostyril und 2,3-Dichlor-5,6-dicyano-benzochinon.

Schmelzpunkt: 157,5–158,5 °C,

Ausbeute: 54% der Theorie.

Beispiel 110

6-[4-(2,5-Dichlorphenylmercapto)-butoxy]-3,4-dihydrocarbostyril

In 54 ml Methanol löst man 2,21 g (0,0315 Mol) Kaliummethylat zusammen mit 5,76 g (0,0315 Mol) 98%igem 2,5-Dichlorthiophenol und fügt dazu 8,94 g (0,03 Mol) 6-(4-Brombutoxy)-3,4-dihydrocarbostyril (Schmelzpunkt: 142–147 °C). Man erhitzt zum Sieden am Rückfluss, wobei zunächst Klarlösung erfolgt. Nach etwa 5 Minuten scheidet sich soviel kristallines Reaktionsprodukt aus, dass das Reaktionsgemisch zu einem kaum mehr rührbaren Kristallbrei erstarrt. Nach einer Stunde kühlt man auf Raumtemperatur ab, saugt ab und kristallisiert aus Äthanol um. Man erhält farblose Kristalle vom Schmelzpunkt 133–134 °C.

Ausbeute: 10,60 g (89,1% der Theorie).

Beispiel 111

6-[4-(2,5-Dichlorphenylsulfinyl)-butoxy]-3,4-dihydrocarbostyril

Man suspendiert in 40 ml Eisessig 5,55 g (0,014 Mol) 6-[4-(2,5-Dichlorphenylmercapto)-butoxy]-3,4-dihydrocarbostyril und versetzt mit 1,19 ml einer 40,06%igen wässrigen Lösung von Wasserstoffperoxid (0,014 Mol), gelöst in 1,5 ml Eisessig, und rührt bei Zimmertemperatur. Die Suspension hellt sich auf und es entsteht eine fast klare Lösung. Nach 70 Stunden haben sich weisse Kristalle abgeschieden. Man saugt ab und kristallisiert aus Äthanol um.

Schmelzpunkt: 185–186 °C,

Ausbeute: 5,43 g (94,1% der Theorie).

Beispiel 112

6-[4-(2,5-Dichlorphenylsulfonyl)-butoxy]-3,4-dihydrocarbostyril

2,97 g (0,0075 Mol) 6-[4-(2,5-Dichlorphenylmercapto)-butoxy]-3,4-dihydrocarbostyril werden in 15 ml eiskalte Ameisensäure eingetragen und mit 1,49 ml 40,08%igem Wasserstoffperoxid (0,0175 Mol) versetzt. Man rührt 2,5 Stunden und verdünnt dann mit der dreifachen Menge Wasser. Die ausgeschiedenen Kristalle werden aus Äthanol umkristallisiert.

Schmelzpunkt: 174,5–175,5 °C,

Ausbeute: 1,99 g (61,9% der Theorie).

Beispiel 113

6-[4-(3,4-Dichlorphenylsulfonyl)-butoxy]-3,4-dihydrocarbostyril

Hergestellt analog Beispiel 112 aus 6-[4-(3,4-Dichlorphenylmercapto)-butoxy]-3,4-dihydrocarbostyril (Schmelzpunkt: 116,5–118 °C) und Wasserstoffperoxid.

Schmelzpunkt: 172–173 °C,

Ausbeute: 96% der Theorie.

Beispiel 114

6-[4-(4-Hydroxy-3,5-di-tert.butyl-phenylmercapto)-butoxy]-3,4-dihydrocarbostyril

Hergestellt analog Beispiel 110 aus 6-(4-Brombutoxy)-3,4-dihydrocarbostyril (Schmelzpunkt: 142–147 °C) und 4-Hydroxy-3,5-di-tert.butylthiophenol.

Schmelzpunkt: 146–147 °C,

Ausbeute: 68,8% der Theorie.

Beispiel 115

6-[4-(4-Hydroxy-3,5-di-tert.-butyl-phenylsulfinyl)-butoxy]-
3,4-dihydrocarbostyril

Hergestellt analog Beispiel 111 aus 6-[4-(4-Hydroxy-3,5-di-tert.-butyl-phenylmercapto)-butoxy]-3,4-dihydrocarbostyril und Wasserstoffperoxid.

Schmelzpunkt: 170–171 °C,

Ausbeute: 80,7% der Theorie.

Beispiel 116

6-[4-(4-Hydroxy-3,5-di-tert.butyl-phenylsulfonyl)-butoxy]-
3,4-dihydrocarbostyril

Hergestellt analog Beispiel 112 aus 6-[4-(4-Hydroxy-3,5-di-tert.-butyl-phenylmercapto)-butoxy]-3,4-dihydrocarbostyril und Wasserstoffperoxid.

Schmelzpunkt: 165–167 °C,

Ausbeute: 97,6% der Theorie.

Beispiel 117

6-[4-(2-Carboxyphenylmercapto)-butoxy]-3,4-dihydrocarbostyril

Hergestellt analog Beispiel 110 aus 6-(4-Brombutoxy)-3,4-dihydrocarbostyril und 2-Thiobenzoesäure.

Schmelzpunkt: 176–179 °C,

Ausbeute: 57,5% der Theorie.

Beispiel 118

6-[4-(2-Carboxy-phenylsulfinyl)-butoxy]-3,4-dihydrocarbostyril

Hergestellt analog Beispiel 111 aus 6-[4-(2-Carboxyphenylmercapto)-butoxy]-3,4-dihydrocarbostyril und Wasserstoffperoxid.

Schmelzpunkt: 194–196 °C,

Ausbeute: 77,2% der Theorie.

Beispiel 119

6-[4-(4-Pyridylsulfinyl)-butoxy]-3,4-dihydrocarbostyril

Hergestellt analog Beispiel 110 aus 6-(4-Brombutoxy)-3,4-dihydrocarbostyril und 4-Mercaptopyridin und anschliessende Oxidation des erhaltenen 6-[4-(4-Pyridylmercapto)-butoxy]-3,4-dihydrocarbostyrils (Schmelzpunkt: 128–133 °C) analog Beispiel 123 mit Wasserstoffperoxid.

Schmelzpunkt: 154 °C,

Ausbeute: 57% der Theorie.

Beispiel 120

6-[4-(4-Pyridylsulfonyl)-butoxy]-3,4-dihydrocarbostyril

Hergestellt analog Beispiel 112 aus 6-[4-(4-Pyridylmercapto)-butoxy]-3,4-dihydrocarbostyril und Wasserstoffperoxid.

Schmelzpunkt: 179–183 °C,

Ausbeute: 35,9% der Theorie.

Beispiel 121

6-(3-Benzylmercapto-propoxy)-3,4-dihydrocarbostyril

Hergestellt analog Beispiel 110 aus 6-(3-Brompropoxy)-3,4-dihydrocarbostyril (Schmelzpunkt: 142–147 °C) und Benzylmercaptan.

Schmelzpunkt: 97,5–99 °C,

Ausbeute: 58% der Theorie.

Beispiel 122

6-(3-Benzylsulfinyl-propoxy)-3,4-dihydrocarbostyril

Hergestellt analog Beispiel 111 aus 6-(3-Benzylmercapto)-3,4-dihydrocarbostyril und Wasserstoffperoxid.

Schmelzpunkt: 144,5–147 °C,

Ausbeute: 51% der Theorie.

Beispiel 123

6-[4-(2-Pyridyl-sulfonyl)-butoxy]-carbostyril

Hergestellt analog Beispiel 112 aus 6-[4-(2-Pyridylsulfinyl)-butoxy]-carbostyril und Wasserstoffperoxid.

Schmelzpunkt: 179–180 °C,

Ausbeute: 65,8% der Theorie.

Beispiel 124

4-Methyl-6-(4-phenylmercapto-butoxy)-carbostyril

5,25 g (0,03 Mol) 4-Methyl-6-hydroxycarbostyril [Schmelzpunkt: 326–330 °C, siehe R.R. Holmes et al. in J. Amer. Chem. Soc. 76, 2404 (1954)], 8,09 g (0,033 Mol) 4-Phenylmercaptobutylbromid und 6,22 g (0,045 Mol) Kaliumcarbonat werden in 70 ml Dimethylsulfoxid bei Raumtemperatur 16 Stunden lang gerührt, danach mit Wasser verdünnt und die ausgeschiedenen Kristalle nach dem Trocknen aus Toluol umkristallisiert.

Schmelzpunkt: 148–150 °C,

Ausbeute: 6,2 g (61,2% der Theorie).

Beispiel 125

4-Methyl-6-(4-phenylsulfinyl-butoxy)-carbostyril

Hergestellt analog Beispiel 124 aus 4-Methyl-6-hydroxycarbostyril (Schmelzpunkt: 326–330 °C) und 4-Phenylsulfinylbutylbromid.

Schmelzpunkt: 167–168 °C,

Ausbeute: 47,3% der Theorie.

Beispiel 126

4-Methyl-6-(4-phenylsulfonyl-butoxy)-carbostyril

Hergestellt analog Beispiel 124 aus 4-Methyl-6-hydroxycarbostyril (Schmelzpunkt: 326–330 °C) und 4-Phenylsulfonylbutylbromid.

Schmelzpunkt: 217–219 °C,

Ausbeute: 66,6% der Theorie.

Beispiel 127

4-Methyl-6-[4-(2-pyridylmercapto)-butoxy]-carbostyril

Hergestellt analog Beispiel 110 aus 4-Methyl-6-(4-brombutoxy)-carbostyril (Schmelzpunkt: 217–219 °C) und 2-Mercaptopyridin.

Schmelzpunkt: 149–151 °C,

Ausbeute: 85,7% der Theorie.

Beispiel 128

4-Methyl-6-[4-(2-pyridylsulfinyl)-butoxy]-carbostyril

Hergestellt analog Beispiel 111 aus 4-Methyl-6-[4-(2-pyridylmercapto)-butoxy]-carbostyril und Wasserstoffperoxid.

Schmelzpunkt: 167–169 °C,

Ausbeute: 61,8% der Theorie.

Beispiel 129

4-Methyl-6-[4-(2-pyridylsulfonyl)-butoxy]-carbostyril

Hergestellt analog Beispiel 112 aus 4-Methyl-6-[4-(2-pyridylmercapto)-butoxy]-carbostyril und Wasserstoffperoxid.

Schmelzpunkt: 195–197 °C,

Ausbeute: 29,5% der Theorie.

Beispiel 130

4-Methyl-6-[4-(2-chinolylmercapto)-butoxy]-carbostyril

Hergestellt analog Beispiel 110 aus 4-Methyl-6-(4-brombutoxy)-carbostyril und 2-Mercaptochinolin.

Schmelzpunkt: 162–163 °C,

Ausbeute: 81,9% der Theorie.

Beispiel 131

4-Methyl-6-[4-(2-chinolylsulfinyl)-butoxy]-carbostyril

Hergestellt analog Beispiel 111 aus 4-Methyl-6-[4-(2-chinolylmercapto)-butoxy]-carbostyril und Wasserstoffperoxid.

Schmelzpunkt: 189–190 °C,

Ausbeute: 47,5% der Theorie.

Beispiel 132

4-Methyl-6-[4-(2-chinolylsulfonyl)-butoxy]-carbostyril

Hergestellt analog Beispiel 112 aus 4-Methyl-6-[4-(2-chinolylmercapto)-butoxy]-carbostyril und Wasserstoffperoxid.

Schmelzpunkt: 199–203 °C,

Ausbeute: 31,5% der Theorie.

Beispiel 133

4-Methyl-6-[4-(4-biphenylylsulfinyl)-butoxy]-carbostyril

Hergestellt analog Beispiel 111 aus 4-Methyl-6-[4-(4-biphenylylmercapto)-butoxy]-carbostyril (Schmelzpunkt: 174–176C) und Wasserstoffperoxid.

Schmelzpunkt: 161–162 °C,

Ausbeute: 59% der Theorie.

Beispiel 134
6-[4-(4-Chlorphenylmercapto)-butoxy]-carbostyril
Hergestellt analog Beispiel 110 aus 6-(4-Chlorbutoxy)-carbostyril (Schmelzpunkt: 206–208 °C) und 4-Chlorthiophenol.
Schmelzpunkt: 168–170 °C,
Ausbeute: 85% der Theorie.

Beispiel 135
6-[4-(4-Chlorphenylsulfinyl)-butoxy]-carbostyril
Hergestellt analog Beispiel 111 aus 6-[4-(4-Chlorphenylmercapto)-butoxy]-carbostyril und Wasserstoffperoxid.
Schmelzpunkt: 157–158 °C,
Ausbeute: 81% der Theorie.

Beispiel 136
6-[4-(4-Chlorphenylsulfonyl)-butoxy]-carbostyril
Hergestellt analog Beispiel 112 aus 6-[4-(4-Chlorphenylmercapto)-butoxy]-carbostyril und Wasserstoffperoxid.
Schmelzpunkt: 197–199 °C,
Ausbeute: 99% der Theorie.

Beispiel 137
6-[4-(3,4-Dichlorphenylmercapto)-butoxy]-carbostyril
Hergestellt analog Beispiel 110 aus 6-(4-Brombutoxy)-carbostyril (Schmelzpunkt: 198–199 °C) und 3,4-Dichlorthiophenol.
Schmelzpunkt: 149–152 °C,
Ausbeute: 60% der Theorie.

Beispiel 138
6-[4-(3,4-Dichlorphenylsulfinyl)-butoxy]-carbostyril
Hergestellt analog Beispiel 111 aus 6-[4-(3,4-Dichlorphenylmercapto)-butoxy]-carbostyril und Wasserstoffperoxid.
Schmelzpunkt: 191–196 °C,
Ausbeute: 87% der Theorie.

Beispiel 139
6-[4-(3,4-Dichlorphenylsulfonyl)-butoxy]-carbostyril
Hergestellt analog Beispiel 112 aus 6-[4-(3,4-Dichlorphenylmercapto)-butoxy]-carbostyril und Wasserstoffperoxid.
Schmelzpunkt: 188–190 °C,
Ausbeute: 83% der Theorie.

Beispiel 140
6-[4-(2,5-Dichlorphenylmercapto)-butoxy]-carbostyril
Hergestellt analog Beispiel 110 aus 6-(4-Brombutoxy)-carbostyril und 2,5-Dichlorthiophenol.
Schmelzpunkt: 175–176 °C,
Ausbeute: 85% der Theorie.

Beispiel 141
6-[4-(2,5-Dichlorphenylsulfinyl)-butoxy]-carbostyril
Hergestellt analog Beispiel 111 aus 6-[4-(2,5-Dichlorphenylmercapto)-butoxy]-carbostyril und Wasserstoffperoxid.
Schmelzpunkt: 200–202 °C,
Ausbeute: 75% der Theorie.

Beispiel 142
6-[4-(2,5-Dichlorphenylsulfonyl)-butoxy]-carbostyril
Hergestellt analog Beispiel 112 aus 6-[4-(2,5-Dichlorphenylmercapto)-butoxy]-carbostyril und Wasserstoffperoxid in Ameisensäure.
Schmelzpunkt: 203–205 °C,
Ausbeute: 79% der Theorie.

Beispiel 143
6-[4-(4-Fluorphenylmercapto)-butoxy]-carbostyril
Hergestellt analog Beispiel 110 aus 6-(4-Brombutoxy)-carbostyril und 4-Fluorthiophenol.
Schmelzpunkt: 149–150 °C,
Ausbeute: 85% der Theorie.

Beispiel 144
6-[4-(4-Fluorphenylsulfinyl)-butoxy]-carbostyril
Hergestellt analog Beispiel 111 aus 6-[4-(4-Fluorphenylmercapto)-butoxy]-carbostyril und Wasserstoffperoxid.
Schmelzpunkt: 164–165 °C,
Ausbeute: 50% der Theorie.

Beispiel 145
6-[4-(4-Fluorphenylsulfonyl)-butoxy]-carbostyril
Hergestellt analog Beispiel 112 aus 6-[4-(4-Fluorphenylmercapto)-butoxy]-carbostyril und Wasserstoffperoxid in Ameisensäure.
Schmelzpunkt: 209–211 °C,
Ausbeute: 59% der Theorie.

Beispiel 146
6-[4-(4-Hydroxy-3,5-di-tert.butyl-phenylmercapto)-butoxy]-carbostyril
Hergestellt analog Beispiel 110 aus 6-(4-Brombutoxy)-carbostyril (Schmelzpunkt: 198–199 °C) und 4-Hydroxy-3,5-di-tert.butyl-thiophenol (Schmelzpunkt: 84,5–86,0 °C).
Schmelzpunkt: 172–173 °C,
Ausbeute: 77% der Theorie.

Beispiel 147
6-[4-(4-Hydroxy-3,5-di-tert.butylphenylsulfinyl)-butoxy]-carbostyril
Hergestellt analog Beispiel 111 aus 6-[4-(4-Hydroxy-3,5-di-tert.-butylphenylmercapto)-butoxy]-carbostyril und Wasserstoffperoxid.
Schmelzpunkt: 192–194 °C,
Ausbeute: 83% der Theorie.

Beispiel 148
6-[4-(4-Hydroxy-3,5-di-tert.butylphenylsulfonyl)-butoxy]-carbostyril
Hergestellt analog Beispiel 112 aus 6-[4-(4-Hydroxy-3,5-di-tert.butylphenylmercapto)-butoxy]-carbostyril und Wasserstoffperoxid in Ameisensäure.
Schmelzpunkt: 242–244 °C,
Ausbeute: 92% der Theorie.

Beispiel 149

6-[4-(4-Biphenylyl-mercapto)-butoxy]-carbostyril

Hergestellt analog Beispiel 110 aus 6-(4-Brombutoxy)-carbostyril (Schmelzpunkt: 198–199 °C) und 4-Biphenylmercaptan.

Schmelzpunkt: 191–192 °C,
Ausbeute: 82% der Theorie.

Beispiel 150

6-[4-(4-Biphenylylsulfinyl)-butoxy]-carbostyril

Hergestellt analog Beispiel 111 aus 6-[4-(4-Biphenylylmercapto)-butoxy]-carbostyril und Wasserstoffperoxid.

Schmelzpunkt: 196–197 °C,
Ausbeute: 80% der Theorie.

Beispiel 151

6-[4-(4-Biphenylyl-sulfonyl)-butoxy]-carbostyril

Hergestellt analog Beispiel 112 aus 6-[4-(4-Biphenylyl-mercapto)-butoxy]-carbostyril und Wasserstoffperoxid.

Schmelzpunkt: 213–215 °C,
Ausbeute: 73% der Theorie.

Beispiel 152

6-[4-(4-Nitro-phenylmercapto)-butoxy]-carbostyril

Hergestellt analog Beispiel 110 aus 6-(4-Brombutoxy)-carbostyril (Schmelzpunkt: 198–199 °C) und 4-Nitrothiophenol.

Schmelzpunkt: 184–185 °C,
Ausbeute: 96% der Theorie.

Beispiel 153

6-[4-(4-Nitrophenylsulfinyl)-butoxy]-carbostyril

Hergestellt analog Beispiel 111 aus 6-[4-(4-Nitrophenylmercapto)-butoxy]-carbostyril und Wasserstoffperoxid.

Schmelzpunkt: 183–184 °C,
Ausbeute: 77% der Theorie.

Beispiel 154

6-[4-(4-Nitrophenylsulfonyl)-butoxy]-carbostyril

Hergestellt analog Beispiel 112 aus 6-[4-(4-Nitrophenylsulfinyl)-butoxy]-carbostyril und Wasserstoffperoxid in Ameisensäure.

Schmelzpunkt: 230–232 °C,
Ausbeute: 70% der Theorie.

Beispiel 155

6-[4-(2-Chinolylmercapto)-butoxy]-carbostyril

Hergestellt analog Beispiel 110 aus 6-(4-Brombutoxy)-carbostyril (Schmelzpunkt: 198–199 °C) und 2-Mercaptochinolin.

Schmelzpunkt: 132 °C,
Ausbeute: 99% der Theorie.

Beispiel 156

6-[4-(2-Chinolylsulfinyl)-butoxy]-carbostyril

Hergestellt analog Beispiel 111 aus 6-[4-(2-Chinolylmercapto)-butoxy]-carbostyril und Wasserstoffperoxid.

Schmelzpunkt: 161–162 °C,
Ausbeute: 66% der Theorie.

Beispiel 157

6-[4-(2-Chinolylsulfonyl)-butoxy]-carbostyril

Hergestellt analog Beispiel 112 aus 6-[4-(2-Chinolylsulfinyl)-butoxy]-carbostyril und Wasserstoffperoxid in Ameisensäure.

Schmelzpunkt: 197–198 °C,
Ausbeute: 58% der Theorie.

Beispiel 158

6-[2-(Phenylsulfinylmethyl)-benzyloxy]-3,4-dihydrocarbostyril

Eine Mischung aus 67,1 g Phthalid, 51,3 ml Thiophenol, 35,1 g Kaliummethylat und 250 ml Methanol wird unter Rückfluss erhitzt. Anschliessend wird die erhaltene 2-Phenylmercaptomethylbenzoesäure (Ausbeute: 78% der Theorie, Schmelzpunkt: 108–110 °C) mit Methanol/Thionylchlorid durch Stehenlassen bei –40 °C verestert. Nach anschliessendem Stehen über Nacht bei Raumtemperatur erhält man 2-Phenylmercaptomethyl-benzoesäuremethylester (Ausbeute: 89% der Theorie, $Kp._{0,07}$:145 °C), welcher durch Reduktion mit Lithiumaluminiumhydrid in Diäthyläther in das 2-Phenylmercaptomethyl-phenyl-carbinol (Ausbeute: 97% der Theorie, Schmelzpunkt: 64–65 °C) übergeführt wird.

Diese Verbindung wird mit p-Toluolsulfochlorid zu 2-Phenylmercaptomethyl-phenylcarbinol-p-toluolsulfosäureester umgesetzt. (Dünnschichtchromatogramm: Kieselgel; Laufmittel: Chloroform/Essigester = 1 : 1; $R_f$ = 0,8. Ausbeute: 55% der Theorie). Dieser Ester wird analog Beispiel 125 mit 6-Hydroxy-3,4-dihydrocarbostyril umgesetzt zu 6-[2-(Phenylmercaptomethyl)-benzyloxy]-3,4-dihydrocarbostyril (Dünnschichtchromatogramm: Kieselgel; Laufmittel: Chloroform/Essigester = 1 : 1, $R_f$ = 0,35; Ausbeute: 64% der Theorie).

Diese Substanz wird analog Beispiel 111 mit Wasserstoffperoxid zu 6-[2-(Phenylsulfinylmethyl)-benzyloxy]-3,4-dihydrocarbostyril oxidiert.

Schmelzpunkt: 133–135 °C,
Ausbeute: 64% der Theorie.

Beispiel 159

6-[4-(Phenylmercaptomethyl)-benzyloxy]-3,4-dihydrocarbostyril

In Dimethylsulfoxid werden in Gegenwart von überschüssigem Kaliumcarbonat p-Xylylendichlorid und Thiophenol im Molverhältnis 1 : 1 bei Raumtemperatur zu 4-Phenylmercaptomethylbenzylchlorid (Kontrolle: Dünnschichtchromatogramm) umgesetzt, welches ohne Isolation in der Reaktionslösung weiter mit 6-Hydroxy-3,4-dihydrocarbostyril analog Beispiel 124 umgesetzt wird.

Schmelzpunkt: 139–141 °C,
Ausbeute: 52% der Theorie.

Beispiel 160

6-[4-(Phenylsulfinylmethyl)-benzyloxy]-3,4-dihydrocarbostyril

Hergestellt analog Beispiel 111 aus 6-[4-(Phenylmercaptomethyl)-benzyloxy]-3,4-dihydrocarbostyril und Wasserstoffperoxid.
Schmelzpunkt: 179–181 °C,
Ausbeute: 61% der Theorie.

Beispiel 161
6-(4-Cyclohexylmercapto-butoxy)-carbostyril
Hergestellt analog Beispiel 110 aus 6-(4-Brombutoxy)-carbostyril (Schmelzpunkt: 198–199 °C) und Cyclohexylmercaptan.
Schmelzpunkt: 153–159 °C,
Ausbeute: 89% der Theorie.

Beispiel 162
6-(4-Cyclohexylsulfinyl-butoxy)-carbostyril
Hergestellt analog Beispiel 111 aus 6-(4-Cyclohexylmercapto-butoxy)-carbostyril und Wasserstoffperoxid.
Schmelzpunkt: 169–170 °C,
Ausbeute: 57% der Theorie.

Beispiel 163
6-[4-(4-Bromphenylmercapto)-butoxy]-carbostyril
Hergestellt analog Beispiel 110 aus 6-(4-Brombutoxy)-carbostyril (Schmelzpunkt: 198–199 °C) und 4-Bromthiophenol.
Schmelzpunkt: 156–158 °C,
Ausbeute: 53% der Theorie.

Beispiel 164
6-[4-(4-Bromphenylsulfinyl)-butoxy]-carbostyril
Hergestellt analog Beispiel 111 aus 6-[4-(4-Bromphenylmercapto)-butoxy]-carbostyril und Wasserstoffperoxid.
Schmelzpunkt: 168–170 °C,
Ausbeute: 65% der Theorie.

Beispiel 165
6-[4-(3-Methyl-4-brom-phenylmercapto)-butoxy]-carbostyril
Hergestellt analog Beispiel 110 aus 6-(4-Brombutoxy)-carbostyril (Schmelzpunkt: 189–199 °C) und 3-Methyl-4-brom-thiophenol.
Schmelzpunkt: 167–169 °C,
Ausbeute: 76% der Theorie.

Beispiel 166
6-[4-(3-Methyl-4-brom-phenylsulfinyl)-butoxy]-carbostyril
Hergestellt analog Beispiel 111 aus 6-[4-(3-Methyl-4-brom-phenylmercapto)-butoxy]-carbostyril und Wasserstoffperoxid.
Schmelzpunkt: 169–172 °C,
Ausbeute: 79% der Theorie.

Beispiel 167
6-[4-(3-Methyl-4-brom-phenylsulfonyl)-butoxy]-carbostyril
Hergestellt analog Beispiel 112 aus 6-[4-(3-Methyl-4-brom-phenyl-mercapto)-butoxy]-carbostyril und Wasserstoffperoxid.
Schmelzpunkt: 163–167 °C,
Ausbeute: 57% der Theorie.

Beispiel 168
6-[4-(1,2,4-Triazol-3-yl-mercapto)-butoxy]-carbostyril
Hergestellt analog Beispiel 110 aus 6-(4-Brombutoxy)-carbostyril und 3-Mercapto-1,2,4-triazol.
Ausbeute: 82% der Theorie.

Beispiel 169
6-[4-(2,4,5-Trichlorphenylmercapto)-butoxy]-carbostyril
Hergestellt analog Beispiel 110 aus 6-(4-Brombutoxy)-carbostyril (Schmelzpunkt: 198–199 °C) und 2,4,5-Trichlorthiophenol.
Schmelzpunkt: 177–178 °C,
Ausbeute: 83% der Theorie.

Beispiel 170
6-[4-(2,4,5-Trichlorphenylsulfinyl)-butoxy]-carbostyril
Hergestellt analog Beispiel 111 aus 6-[4-(2,4,5-Trichlorphenylmercapto)-butoxy]-carbostyril und Wasserstoffperoxid.
Schmelzpunkt: 206–208 °C,
Ausbeute: 98% der Theorie.

Beispiel 171
6-[4-(3,5-Dibrom-3-amino-phenylmercapto)-butoxy]-3,4-dihydrocarbostyril
Hergestellt analog Beispiel 110 aus 6-(4-Brombutoxy)-3,4-dihydrocarbostyril (Schmelzpunkt: 142–147 °C) und 3,5-Dibrom-4-aminothiophenol.
Schmelzpunkt: 90–92 °C,
Ausbeute: 89% der Theorie.

Beispiel 172
6-[4-(3,5-Dibrom-4-amino-phenyl-sulfinyl)-butoxy]-3,4-dihydrocarbostyril
Hergestellt analog Beispiel 111 aus 6-[4-(3,5-Dibrom-4-aminophenyl-mercapto)-butoxy]-3,4-dihydrocarbostyril und Wasserstoffperoxid.
Schmelzpunkt: 144–146 °C,
Ausbeute: 76% der Theorie.

Beispiel 173
6-[4-(3,5-Dibrom-4-amino-phenylsulfonyl)-butoxy]-3,4-dihydrocarbostyril
Hergestellt analog Beispiel 112 aus 6-[4-(3,5-Dibrom-4-aminophenyl-sulfinyl)-butoxy]-3,4-dihydrocarbostyril und Wasserstoffperoxid.
Schmelzpunkt: 157–159 °C,
Ausbeute: 87% der Theorie.

Beispiel 174
6-[4-(3,5-Dibrom-4-amino-phenylmercapto)-butoxy]-carbostyril
Hergestellt analog Beispiel 110 aus 6-(4-Brombutoxy)-carbostyril (Schmelzpunkt: 198–199 °C) und 3,5-Dibrom-4-amino-thiophenol.
Schmelzpunkt: 153–155 °C,
Ausbeute: 86% der Theorie.

Beispiel 175
6-[4-(3,5-Dibrom-4-amino-phenylsulfinyl)-butoxy]-carbostyril

Hergestellt analog Beispiel 111 aus 6-[4-(3,5-Dibrom-4-aminophenyl-mercapto)-butoxy]-carbostyril und Wasserstoffperoxid.
Schmelzpunkt: 205–207 °C,
Ausbeute: 79% der Theorie.

Beispiel 176
6-[4-(3,5-Dibrom-4-amino-phenylsulfonyl)-butoxy]-carbostyril
Hergestellt analog Beispiel 112 aus 6-[4-(3,5-Dibrom-4-amino-phenyl-mercapto)-butoxy]-carbostyril und Wasserstoffperoxid in Ameisensäure.
Schmelzpunkt: 238–241 °C,
Ausbeute: 87% der Theorie.

Beispiel 177
6-[4-(4-Brom-3-methyl-phenylmercapto)-butoxy]-3,4-dihydro-carbostyril
Hergestellt analog Beispiel 110 aus 6-(4-Brombutoxy)-3,4-dihydro-carbostyril (Schmelzpunkt: 142–147 °C) und 4-Brom-3-methyl-thiophenol.
Schmelzpunkt: 104–109 °C,
Ausbeute: 81% der Theorie.

Beispiel 178
6-[4-(4-Brom-3-methyl-phenylsulfinyl)-butoxy]-3,4-dihydrocarbostyril
Hergestellt analog Beispiel 111 aus 6-[4-(4-Brom-3-methyl-phenylmercapto)-butoxy]-3,4-dihydrocarbostyril und Wasserstoffperoxid.
Schmelzpunkt: 129–130 °C,
Ausbeute: 75% der Theorie.

Beispiel 179
6-[4-(2,5-Dibrom-phenylmercapto)-butoxy]-3,4-dihydrocarbostyril
Hergestellt analog Beispiel 110 aus 6-(4-Brombutoxy)-3,4-dihydrocarbostyril (Schmelzpunkt: 142–147 °C) und 2,5-Dibromthiophenol.
Schmelzpunkt: 127–129 °C,
Ausbeute: 75% der Theorie.

Beispiel 180
6-[4-(2,5-Dibrom-phenylsulfinyl)-butoxy]-3,4-dihydrocarbostyril
Hergestellt analog Beispiel 111 aus 6-[4-(2,5-Dibrom-phenylmercapto)-butoxy]-3,4-dihydrocarbostyril und Wasserstoffperoxid.
Schmelzpunkt: 182–184 °C,
Ausbeute: 84% der Theorie.

Beispiel 181
6-[4-(2,5-Dibrom-phenylmercapto)-butoxy]-carbostyril
Hergestellt analog Beispiel 110 aus 6-(4-Brombutoxy)-carbostyril (Schmelzpunkt: 198–199 °C) und 2,5-Dibrom-thiophenol.
Schmelzpunkt: 178–185 °C,
Ausbeute: 67% der Theorie.

Beispiel 182
6-[4-(2,5-Dibrom-phenylsulfinyl)-butoxy]-carbostyril
Hergestellt analog Beispiel 111 aus 6-[4-(2,5-Dibrom-phenylmercapto)-butoxy]-carbostyril und Wasserstoffperoxid.
Schmelzpunkt: 187–189 °C,
Ausbeute: 45% der Theorie.

Beispiel 183
6-[3-(3,4-Dichlor-phenylmercapto)-propoxy]-3,4-dihydrocarbostyril
Hergestellt analog Beispiel 110 aus 6-(3-Brompropoxy)-3,4-dihydrocarbostyril (Schmelzpunkt: 111–118 °C) und 3,4-Dichlor-thiophenol.
Schmelzpunkt: 106–107 °C,
Ausbeute: 76% der Theorie.

Beispiel 184
6-[3-(3,4-Dichlor-phenylsulfinyl)-propoxy]-3,4-dihydrocarbostyril
Hergestellt analog Beispiel 111 aus 6-[3-(3,4-Dichlor-phenylmercapto)-propoxy]-3,4-dihydrocarbostyril und Wasserstoffperoxid.
Schmelzpunkt: 170–172 °C,
Ausbeute: 84% der Theorie.

Beispiel 185
6-[4-(4-Cyclohexyl-phenylmercapto)-butoxy]-3,4-dihydrocarbostyril
Hergestellt analog Beispiel 110 aus 6-(4-Brombutoxy)-3,4-dihydrocarbostyril (Schmelzpunkt: 142–147 °C) und 4-Cyclohexyl-thiophenol.
Schmelzpunkt: 118–120 °C,
Ausbeute: 68% der Theorie.

Beispiel 186
6-[4-(4-Cyclohexyl-phenylsulfinyl)-butoxy]-3,4-dihydrocarbostyril
Hergestellt analog Beispiel 111 aus 6-[4-(4-Cyclohexyl-phenylmercapto)-butoxy]-3,4-dihydrocarbostyril und Wasserstoffperoxid.
Schmelzpunkt: 155–157 °C,
Ausbeute: 65% der Theorie.

Beispiel 187
6-[4-(4-Cyclohexyl-phenylsulfonyl)-butoxy]-3,4-dihydrocarbostyril
Hergestellt analog Beispiel 112 aus 6-[4-(4-Cyclohexyl-phenylmercapto)-butoxy]-3,4-dihydrocarbostyril und Wasserstoffperoxid.
Schmelzpunkt: 172–174 °C,
Ausbeute: 52% der Theorie.

Beispiel 188
6-[4-(4-Cyclohexyl-phenylmercapto)-butoxy]-carbostyril
Hergestellt analog Beispiel 110 aus 6-(4-Brombutoxy)-carbostyril (Schmelzpunkt: 188–189 °C) und 4-Cyclohexylthiophenol.
Schmelzpunkt: 165–167 °C,
Ausbeute: 64% der Theorie.

Beispiel 189
6-[4-(4-Cyclohexyl-phenylsulfinyl)-butoxy]-carbostyril

Hergestellt analog Beispiel 111 aus 6-[4-(4-Cyclohexyl-phenylmercapto)-butoxy]-carbostyril und Wasserstoffperoxid.

Schmelzpunkt: 188–190 °C,

Ausbeute: 64% der Theorie.

Beispiel 190

6-[4-(4-Cyclohexyl-phenylsulfonyl)-butoxy]-carbostyril

Hergestellt analog Beispiel 112 aus 6-[4-(4-Cyclohexyl-phenylmercapto)-butoxy]-carbostyril und Wasserstoffperoxid.

Schmelzpunkt: 185–186 °C,

Ausbeute: 87% der Theorie.

Beispiel 191

6-[4-(4-tert.Butyl-phenylmercapto)-butoxy]-3,4-dihydrocarbostyril

Hergestellt analog Beispiel 110 aus 6-(4-Brombutoxy)-3,4-dihydrocarbostyril (Schmelzpunkt: 142–147 °C) und 4-tert.Butylthiophenol.

Schmelzpunkt: 126–127 °C,

Ausbeute: 86% der Theorie.

Beispiel 192

6-[4-(4-tert.Butyl-phenylsulfinyl)-butoxy]-3,4-dihydrocarbostyril

Hergestellt analog Beispiel 111 aus 6-[4-(4-tert.-Butyl-phenylmercapto)-butoxy]-3,4-dihydrocarbostyril und Wasserstoffperoxid.

Schmelzpunkt: 121–123 °C,

Ausbeute: 67% der Theorie.

Beispiel 193

6-[4-(4-tert.Butyl-phenylsulfonyl)-butoxy]-3,4-dihydrocarbostyril

Hergestellt analog Beispiel 112 aus 6-[4-(4-tert.-Butyl-phenylmercapto)-butoxy]-3,4-dihydrocarbostyril und Wasserstoffperoxid.

Schmelzpunkt: 198–200 °C,

Ausbeute: 83% der Theorie.

Beispiel 194

6-[4-(4-tert.Butyl-phenylmercapto)-butoxy]-carbostyril

(Ausgangsverbindung für Beispiel 192 und 193) Hergestellt analog Beispiel 110 aus 6-(4-Brombutoxy)-carbostyril (Schmelzpunkt: 198–199 °C) und 4-tert.Butylthiophenol.

Schmelzpunkt: 156–158 °C,

Ausbeute: 63% der Theorie.

Beispiel 195

6-[4-(4-tert.Butyl-phenylsulfinyl)-butoxy]-carbostyril

Hergestellt analog Beispiel 111 aus 6-[4-(4-tert.-Butyl-phenylmercapto)-butoxy]-carbostyril und Wasserstoffperoxid.

Schmelzpunkt: 164–166 °C,

Ausbeute: 74% der Theorie.

Beispiel 196

6-[4-(4-tert.Butyl-phenylsulfonyl)-butoxy]-carbostyril

Hergestellt analog Beispiel 111 aus 6-[4-(4-tert.-Butyl-phenylmercapto)-butoxy]-carbostyril und Wasserstoffperoxid.

Schmelzpunkt: 203–205 °C,

Ausbeute: 56% der Theorie.

Beispiel 197

6-[4-(2-Chinolylsulfinyl)-butoxy]-3,4-dihydrocarbostyril

Hergestellt analog Beispiel 111 aus 6-[4-(2-Chinolylmercapto)-butoxy]-3,4-dihydrocarbostyril und Wasserstoffperoxid.

Schmelzpunkt: 154–157 °C,

Ausbeute: 79% der Theorie.

Beispiel 198

6-[4-(2-Chinolylsulfonyl)-butoxy]-3,4-dihydrocarbostyril

Hergestellt analog Beispiel 112 aus 6-[4-(2-Chinolylmercapto)-butoxy]-3,4-dihydrocarbostyril und Wasserstoffperoxid.

$R_f$-Wert: 0,50 (Kieselgelleuchtstoffplatte; Laufmittel: Benzol/Äthanol/konz. Ammoniak = 75:25:2),

Ausbeute: 72% der Theorie.

Beispiel 199

6-[2-(N-Methyl-N-cyclohexyl-carbamidomethylmercapto)-äthoxy]-3,4-dihydrocarbostyril

Hergestellt analog Beispiel 110 aus 6-(2-Chloräthoxy)-3,4-dihydrocarbostyril (Schmelzpunkt: 152,5–153,5 °C) und N-Methyl-N-cyclohexyl-thioglycolsäure-amid.

$R_f$-Wert: 0,46 (Kieselgelleuchtstoffplatte; Laufmittel: Äthylenchlorid/Methanol = 95:5).

Ausbeute: 63% der Theorie.

Beispiel 200

6-[4-(N-Methyl-N-cyclohexyl-carbamidomethylsulfinyl)-äthoxy]-3,4-dihydrocarbostyril

Hergestellt analog Beispiel 111 aus 6-[2-(N-Methyl-N-cyclohexyl-carbamidomethylmercapto)-äthoxy]-3,4-dihydrocarbostyril und Wasserstoffperoxid.

$R_f$-Wert: 0,34 (Kieselgelleuchtstoffplatte; Laufmittel: Äthylenchlorid/Methanol = 95:5).

Schmelzpunkt: 143–146 °C,

Ausbeute: 48% der Theorie.

Beispiel 201

6-[2-(N-Methyl-N-cyclohexyl-carbamidomethylsulfonyl)-äthoxy]-3,4-dihydrocarbostyril

Hergestellt analog Beispiel 112 aus 6-[2-(N-Methyl-N-cyclohexyl-carbamidomethylmercapto)-äthoxy]-3,4-dihydrocarbostyril und Wasserstoffperoxid in Ameisensäure.

$R_f$-Wert: 0,48 (Kieselgelleuchtstoffplatte; Laufmittel: Äthylenchlorid/Methanol = 95:5).

Schmelzpunkt: 110–111 °C,

Ausbeute: 45% der Theorie.

Beispiel 202

6-[2-(N-Methyl-N-cyclohexyl-carbamidomethylmercapto)-äthoxy]-carbostyril

Hergestellt analog Beispiel 110 aus 6-(2-Chloräthoxy)-carbostyril [$R_f$-Wert: 0,30 (Kieselgelleuchtstoffplatte; Laufmittel: Äthylenchlorid/Methanol = 95:5)] und N-Methyl-N-cyclohexyl-thioglykolsäureamid.

$R_f$-Wert: 0,41 (Kieselgelleuchtstoffplatte; Laufmittel: Äthylenchlorid/Methanol = 95:5).

Ausbeute: 62% der Theorie.

Beispiel 203

6-[2-(N-Methyl-N-cyclohexyl-carbamidomethylsulfinyl)-äthoxy]-carbostyril

Hergestellt analog Beispiel 111 aus 6-[2-(N-Methyl-N-cyclohexyl-carbamidomercapto)-äthoxy]-carbostyril und Wasserstoffperoxid.

$R_f$-Wert: 0,027 (Kieselgelleuchtstoffplatte; Laufmittel: Äthylenchlorid/Methanol = 95:5).

Schmelzpunkt: 128–130 °C,

Ausbeute: 65% der Theorie.

Beispiel 204

6-[2-(N-Methyl-N-cyclohexyl-carbamidomethylsulfonyl)-äthoxy]-carbostyril

Hergestellt analog Beispiel 112 aus 6-[2-(N-Methyl-N-cyclohexyl-carbamidomercapto)-äthoxy]-carbostyril und Wasserstoffperoxid.

$R_f$-Wert: 0,39 (Kieselgelleuchtstoffplatte; Laufmittel: Äthylenchlorid/Methanol = 95:5).

Ausbeute: 67% der Theorie.

Beispiel 205

6-[3-(3,4-Dichlorphenylsulfonyl)-propoxy]-3,4-dihydrocarbostyril

Hergestellt analog Beispiel 112 aus 6-[3-(3,4-Dichlorphenylmercapto)-propoxy]-3,4-dihydrocarbostyril und Wasserstoffperoxid.

Schmelzpunkt: 187–188 °C,

Ausbeute: 87% der Theorie.

Beispiel 206

6-[5-(3,4-Dichlorphenylmercapto)-pentoxy]-3,4-dihydrocarbostyril

Hergestellt analog Beispiel 110 aus 6-(5-Brompentoxy)-3,4-dihydrocarbostyril (Schmelzpunkt: 97–98 °C) und 3,4-Dichlorthiophenol.

Schmelzpunkt: 101–104 °C,

Ausbeute: 69% der Theorie.

Beispiel 207

6-[5-(3,4-Dichlorphenylsulfinyl)-pentoxy]-3,4-dihydrocarbostyril

Hergestellt analog Beispiel 111 aus 6-[5-(3,4-Dichlorphenylmercapto)-pentoxy]-3,4-dihydrocarbostyril und Wasserstoffperoxid.

Schmelzpunkt: 165–166 °C,

Ausbeute: 74% der Theorie.

Beispiel 208

6-[5-(3,4-Dichlorphenylsulfonyl)-pentoxy]-3,4-dihydrocarbostyril

Hergestellt analog Beispiel 112 aus 6-[5-(3,4-Dichlorphenylmercapto)-pentoxy]-3,4-dihydrocarbostyril und Wasserstoffperoxid.

Schmelzpunkt: 176–178 °C,

Ausbeute: 65% der Theorie.

Beispiel 209

6-[4-(2-Methyl-4-tert.butyl-phenylmercapto)-butoxy]-3,4-dihydrocarbostyril

Hergestellt analog Beispiel 110 aus 6-(4-Brombutoxy)-3,4-dihydrocarbostyril (Schmelzpunkt: 142–147 °C) und 2-Methyl-4-tert.butyl-thiophenol.

Schmelzpunkt: 81–85 °C,

Ausbeute: 91% der Theorie.

Beispiel 210

6-[4-(2-Methyl-4-tert.butyl-phenylsulfinyl)-butoxy]-3,4-dihydrocarbostyril

Hergestellt analog Beispiel 111 aus 6-[4-(2-Methyl-4-tert.butylphenylmercapto)-butoxy]-3,4-dihydrocarbostyril und Wasserstoffperoxid. Harzige Substanz.

$R_f$-Wert: 0,54 (Kieselgelleuchtstoffplatte; Laufmittel: Äthylenchlorid/Methanol = 95:5).

Beispiel 211

6-[4-(3,5-Dichlor-4-hydroxy-phenylmercapto)-butoxy]-3,4-dihydrocarbostyril

Hergestellt analog Beispiel 110 aus 6-(4-Brombutoxy)-3,4-dihydrocarbostyril (Schmelzpunkt: 142–147 °C) und 3,5-Dichlor-4-hydroxy-thiophenol unter Stickstoff als Schutzgas.

Schmelzpunkt: 110–114 °C,

Ausbeute: 94% der Theorie.

Beispiel 212

5-Brom-6-(4-phenylmercaptobutoxy)-carbostyril

Hergestellt analog Beispiel 110 aus 5-Brom-6-(4-brombutoxy)-carbostyril (hergestellt durch Bromierung von 6-(4-Brombutoxy)-carbostyril) und Thiophenol.

Schmelzpunkt: 209–213 °C,

Ausbeute: 41% der Theorie.

Beispiel 213

5-Nitro-6-(4-phenylmercaptobutoxy)-carbostyril

Hergestellt analog Beispiel 110 aus 5-Nitro-6-(4-brombutoxy)-carbostyril (Schmelzpunkt: 250 °C, hergestellt durch Nitrieren von 6-(4-Brombutoxy)-3,4-dihydrocarbostyril) und Thiophenol.

Schmelzpunkt: 288–230 °C,

Ausbeute: 71% der Theorie.

Beispiel 214

5-Nitro-6-(4-phenylsulfinylbutoxy)-carbostyril

Hergestellt analog Beispiel 111 aus 5-Nitro-6-(4-phenylmercaptobutoxy)-carbostyril und Wasserstoffperoxid.

Schmelzpunkt: 192–194 °C,

Ausbeute: 91% der Theorie.

Beispiel 215

5-Acetamino-6-(4-phenylmercaptobutoxy)-carbostyril

Hergestellt analog Beispiel 110 aus 5-Acetamino-6-(4-brombutoxy)-carbostyril (hergestellt durch Reduktion mit Zink in Essigsäure von 5-Nitro-6-(4-brombutoxy)-carbostyril unter Zusatz von Acetanhydrid) und Thiophenol.

Schmelzpunkt: 238–240 °C,

Ausbeute: 80% der Theorie.

Beispiel 216
5-Acetamino-6-(4-phenylsulfinylbutoxy)-carbostyril

Hergestellt analog Beispiel 111 aus 5-Acetamino-6-(4-phenylmercapto)-butoxy)-carbostyril und Wasserstoffperoxid.

Schmelzpunkt: 213–217 °C,
Ausbeute: 47% der Theorie.

Beispiel 217
5-Brom-6-(4-phenylsulfinylbutoxy)-carbostyril

Hergestellt analog Beispiel 111 aus 5-Brom-6-(4-phenylmercapto-butoxy)-carbostyril und Wasserstoffperoxid.

Schmelzpunkt: 190–191 °C,
Ausbeute: 82% der Theorie.

Beispiel 218
4-Methyl-6-[4-(2-pyridylsulfinyl)-butoxy]-carbostyril

0,170 g (0,0005 Mol) 4-Methyl-6-[4-(2-pyridylmercapto)-butoxy]-carbostyril, gelöst in 5 ml Eisessig, und 0,107 g (0,0005 Mol) Natriummetaperjodat, gelöst in 6 ml Wasser, werden gemischt und bei Raumtemperatur 22 Stunden lang stehen gelassen. Danach wird die hellbraune Lösung mit 20 ml Wasser verdünnt und das Reaktionsprodukt mit Chloroform extrahiert. Man schüttelt den Extrakt einmal mit Natriumbikarbonatlösung und trocknet über wasserfreiem Magnesiumsulfat. Nach dem Abdampfen wird aus Toluol umkristallisiert.

Schmelzpunkt: 166–168 °C,
Ausbeute: 0,04 g (22,5% der Theorie).

Beispiel 219
6-(4-tert.Butylsulfinyl-butoxy)-3,4-dihydrocarbostyril

Hergestellt analog Beispiel 111 aus 6-(4-tert.-Butylmercaptobutoxy)-3,4-dihydrocarbostyril (Schmelzpunkt: 117–118 °C) und Wasserstoffperoxid.

Schmelzpunkt: 126–128 °C,
Ausbeute: 62% der Theorie.

Beispiel 220
6-[4-(3-Hydroxy-pyrid-2-yl-mercapto)-butoxy]-3,4-dihydrocarbostyril

Hergestellt analog Beispiel 110 aus 6-(4-Brombutoxy)-3,4-dihydrocarbostyril (Schmelzpunkt: 142–147 °C) und 3-Hydroxy-2-mercapto-pyridin.

Schmelzpunkt: 211–216 °C,
Ausbeute: 58% der Theorie.

Beispiel 221
6-[4-(1,2,4-Triazol-3-yl-sulfinyl)-butoxy]-carbostyril

Hergestellt analog Beispiel 111 aus 6-[4-(1,2,4-Triazol-3-yl-mercapto)-butoxy]-carbostyril und Wasserstoffperoxid.

$R_f$-Wert: 0,12 (Kieselgelleuchtstoffplatte; Laufmittel: Äthylenchlorid/Methanol = 95:5).

Beispiel 222
6-[4-(1,2,4-Triazol-3-yl-mercapto)-butoxy]-3,4-dihydrocarbostyril

Hergestellt analog Beispiel 110 aus 6-(4-Brombutoxy)-3,4-dihydro-carbostyril (Schmelzpunkt: 142–147 °C) und 3-Mercapto-1,2,4-triazol.

Schmelzpunkt: 152–154 °C,
Ausbeute: 82% der Theorie.

Beispiel 223
6-[4-(1,2,4-Triazol-3-yl-sulfinyl)-butoxy]-3,4-dihydrocarbostyril

Hergestellt analog Beispiel 111 aus 6-[4-(1,2,4-Triazol-3-yl-mercapto)-butoxy]-3,4-dihydrocarbostyril und Wasserstoffperoxid. $R_f$-Wert: 0,18 (Kieselgelleuchtstoffplatte; Laufmittel: Äthylenchlorid/Methanol = 95:5).

Beispiel 224
6-[4-(1,2,4-Triazol-3-yl-sulfonyl)-butoxy]-3,4-dihydrocarbostyril

Hergestellt analog Beispiel 112 aus 6-[4-(1,2,4-Triazol-3-yl-mercapto)-butoxy]-3,4-dihydrocarbostyril und Wasserstoffperoxid.

$R_f$-Wert: 0,22 (Kieselgelleuchtstoffplatte, Laufmittel: Äthylenchlorid/Methanol = 95:5),
Schmelzpunkt: 217–224 °C.

Beispiel 225
6-[4-(2,4,5-Trichlorphenylmercapto)-butoxy]-3,4-dihydrocarbostyril

Hergestellt analog Beispiel 110 aus 6-(4-Brombutoxy)-3,4-dihydrocarbostyril (Schmelzpunkt: 142–147 °C) und 2,4,5-Trichlor-thiophenol.

Schmelzpunkt: 144–145 °C,
Ausbeute: 87% der Theorie.

Beispiel A
Tabletten mit 100 mg 6-[4-(2-Pyridylsulfinyl)-butoxy]-3,4-dihydrocarbostyril

Zusammensetzung:
1 Tablette enthält:

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren:

Wirkstoff, Milchzucker und Stärke werden gemischt und mit einer wässrigen Lösung des Polyvinylpyrrolidons gleichmässig befeuchtet. Nach Siebung der feuchten Masse (2,0 mm-Maschenweite) und Trocknen im Hordentrockenschrank bei 50 °C wird erneut gesiebt (1,5 mm-Maschenweite) und das Schmiermittel zugemischt.

Die pressfertige Mischung wird zu Tabletten verarbeitet.

Tablettengewicht: 220 mg
Durchmesser: 10 mm, biplan mit beidseitiger Facette und einseitiger Teilkerbe.

Beispiel B

Dragées mit 50 mg 6-(4-Phenylsulfinylbutoxy)-3,4-dihydrocarbostyril

1 Dragéekern enthält:

| | |
|---|---|
| Wirkstoff | 50,0 mg |
| Milchzucker | 40,0 mg |
| Maisstärke | 17,0 mg |
| Polyvinylpyrrolidon | 2,0 mg |
| Magnesiumstearat | 1,0 mg |
| | 110,0 mg |

Herstellung:

Das Granulat wurde analog Beispiel A hergestellt. Die pressfertige Mischung wird zu Dragéekernen verarbeitet.

Kerngewicht: 110 mg

Durchmesser: 8 mm, bikonvex.

Die Kerne werden im Dragierkessel nach Isolierung mit einer Polyvinylpyrrolidonschicht und einer gebräuchlichen Zuckerdragiersuspension bis 200 mg überzogen und anschliessend mit reinem Zuckersirup auf 210 mg dragiert.

Beispiel C

Hartgelatine-Kapseln mit 100 mg 6-[4-(2-Pyridylsulfinyl)-butoxy]-3,4-dihydrocarbostyril

1 Kapsel enthält:

| | | |
|---|---|---|
| Wirkstoff | | 100,0 mg |
| Maisstärke getr. | ca. | 130,0 mg |
| Milchzucker pulv. | ca. | 87,0 mg |
| Magnesiumstearat | | 3,0 mg |
| | | 320,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Grösse 1 abgefüllt.

Kapselfüllung: ca. 320 mg

Kapselhülle: Hartgelatine-Kapsel Grösse 1.

Beispiel D

Suppositorien mit 150 mg 6-(4-Phenylsulfinylbutoxy)-3,4-dihydrocarbostyril

1 Zäpfchen enthält:

| | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2000,0 mg |

Herstellung:

Nach dem Aufschmelzen der Suppositorienmasse wird der Wirkstoff darin homogen verteilt und die Schmelze in vorgekühlte Formen gegossen.

Beispiel E

Suspension mit 50 mg 6-[4-(2-Pyridylsulfinyl)-butoxy]-3,4-dihydrocarbostyril pro 5 ml

100 ml Suspension enthalten:

| | | |
|---|---|---|
| Wirkstoff | | 1,0 g |
| Carboxymethylcellulose-Na-Salz | | 0,1 g |
| p-Hydroxybenzoesäuremethylester | | 0,05 g |
| p-Hydroxybenzoesäurepropylester | | 0,01 g |
| Rohrzucker | | 10,0 g |
| Glycerin | | 5,0 g |
| Sorbitlösung 70%ig | | 20,0 g |
| Aroma | | 0,3 g |
| Wasser dest. | ad | 100 ml |

Herstellung:

Dest. Wasser wird auf 70 °C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

**Patentansprüche**

1. Neue Carbostyrilderivate der allgemeinen Formel

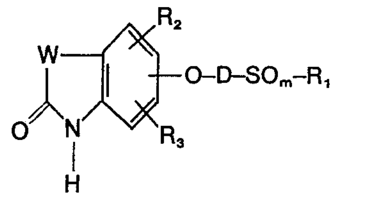

(I),

in der

W eine gegebenenfalls durch eine Methylgruppe substituierte Vinylengruppe oder die Äthylengruppe,

m die Zahl 0, 1 oder 2,

D eine geradkettige oder verzweigte Alkylengruppe mit 2 bis 6 Kohlenstoffatomen, wobei zwischen dem Sauerstoffatom und der $SO_m$-Gruppe mindestens 2 Kohlenstoffatome liegen müssen, oder eine Xylylengruppe,

$R_1$ eine Cycloalkylgruppe mit 3 bis 6 Kohlenstoffatomen, eine Arylgruppe mit 6 bis 10 Kohlenstoffatomen oder eine Aralkylgruppe mit 7 bis 11 Kohlenstoffatomen, wobei die oben aufgeführten aromatischen Kerne durch eine Alkylgruppe mit 1 bis 4 Kohlenstoffatomen, durch eine Hydroxy-, Methoxy-, Amino-, Acetylamino-, Nitro-, Carboxyl-, Cyclohexyl-, Phenylgruppe oder ein Halogenatom monosubstituiert und zusätzlich die oben erwähnten monosubstituierten Phenylgruppen durch Alkylgruppen mit 1 bis 4 Kohlenstoffatomen und/oder Halogenatome mono- oder disubstituiert sein können (wobei die Substituenten des Phenylkerns gleich oder verschieden sein können), eine N-Methyl-cyclohexylamino-carbonylmethyl-, Amino-iminomethylen-, Pyridyl-, Pyridylmethyl-, Furfuryl-, Benzimidazolyl-, Benzthiazolyl-, Pyrimidyl-, Chinolyl-, Chinazolin-4-on-yl-, 4,5-Bis-(p-chlorphenyl)-oxazol-2-yl-, Pyridyl-oxid-, Triazolyl-, Methyl-pyridyl-, Methoxy-pyridyl-, Fluor-pyri-

dyl-, Chlorpyridyl-, Amino-pyridyl-, Acetylamino-pyridyl- oder Triphenylmethylgruppe oder auch eine tert. Butylgruppe, wenn

m die Zahl 1 darstellt,

$R_2$ und $R_3$, die gleich oder verschieden sein können, Wasserstoff- oder Halogenatome, Alkylgruppen mit 1 bis 4 Kohlenstoffatomen, Amino-, Acetylamino- oder Nitrogruppen bedeuten.

2. Neue Verbindungen der allgemeinen Formel I gemäss Anspruch 1, in der

W, D und m wie im Anspruch 1 definiert sind,

$R_1$ eine Cyclohexyl-, Benzyl-, Naphthyl-, Pyridyl-, Pyrimidyl-, 1,2,4-Triazolyl-, Pyridyl-oxid-, Furfuryl-, Triphenylmethyl-, Chinolyl-, Benzimidazolyl-, Benzthiazolyl-, Chinazolin-4-on-yl-, 4,5-bis-(p-Chlorphenyl)-oxazol-2-yl-, N-Methyl-cyclohexylamino-carbonylmethyl- oder Aminoiminomethylgruppe, eine gegebenenfalls durch eine Carboxyl-, Hydroxy-, Methoxy-, Amino-, Acetylamino-, Nitro-, Cyclohexyl- oder Phenylgruppe substituierte Phenylgruppe, eine durch Halogenatome mono- oder disubstituierte Phenylgruppe, eine durch zwei Halogenatome oder durch zwei Alkylgruppen mit 1 bis 4 Kohlenstoffatomen substituierte Hydroxyphenyl-, Halogenphenyl- oder Aminophenylgruppe,

$R_2$ ein Wasserstoff-, Chlor- oder Bromatom, die Methyl-, Amino-, Acetylamino- oder Nitrogruppe und

$R_3$ ein Wasserstoffatom bedeuten.

3. Neue Verbindungen der allgemeinen Formel I gemäss Anspruch 1, in der

W eine gegebenenfalls durch eine Methylgruppe substituierte Vinylengruppe oder die Äthylengruppe,

m die Zahl 0, 1 oder 2,

D eine Alkylengruppe mit 2 bis 5 Kohlenstoffatomen, wobei zwischen dem Sauerstoffatom und der $SO_m$-Gruppe mindestens 2 Kohlenstoffatome liegen müssen,

$R_1$ eine Cyclohexyl-, Phenyl-, Benzyl-, Naphthyl-, Biphenylyl-, Cyclohexylphenyl-, Pyridyl-, Methylphenyl-, Methoxyphenyl-, Fluorphenyl-, Chlorphenyl-, Dichlorphenyl-, Trichlorphenyl-, Bromphenyl-, Dibromphenyl-, Brom-methylphenyl-, Aminodibromphenyl- oder Hydroxy-di-tert.butylphenylgruppe,

$R_2$ und $R_3$ je ein Wasserstoffatom bedeuten.

4. Neue Verbindungen der allgemeinen Formel I gemäss Anspruch 1, in der

W die Äthylen-, Vinylen- oder 2-Methylvinylengruppe,

m die Zahl 1 oder 2,

$R_2$ und $R_3$ je ein Wasserstoffatom,

$R_1$ die Cyclohexyl-, Phenyl-, Benzyl-, Naphthyl-(2)-, 2-Methoxyphenyl-, 4-Chlorphenyl-, 3,4-Dichlorphenyl-, 2,5-Dichlorphenyl-, 4-Amino-3,5-dibromphenyl-, 4-Hydroxy-3,5-di-tert.butylphenyl- oder Pyridyl-(2)-gruppe und

D die Äthylen-, n-Propylen- oder n-Butylengruppe bedeuten.

5. 6-(4-Phenylsulfinylbutoxy)-3,4-dihydrocarbostyril.

6. 6-[4-(3,4-Dichlorphenylsulfinyl)-butoxy]-3,4-dihydrocarbostyril.

7. 6-[4-(2-Pyridylsulfonyl)-butoxy]-carbostyril.

8. Arzneimittel, enthaltend eine Verbindung gemäss den Ansprüchen 1 bis 7 neben einem oder mehreren inerten Trägerstoffen oder Verdünnungsmitteln.

9. Verbindungen gemäss den Ansprüchen 1 bis 7 zur Herstellung eines Heilmittels gegen thrombo-embolische Krankheiten auf nichtchemischem Wege.

10. Verfahren zur Herstellung von neuen Carbostyrilderivaten gemäss den Ansprüchen 1 bis 7, dadurch gekennzeichnet, dass

a) eine Hydroxyverbindung der allgemeinen Formel

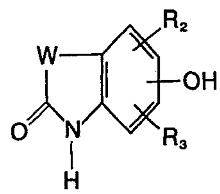 (II),

in der

$R_2$, $R_3$ und W wie eingangs definiert sind, oder deren Salze mit anorganischen oder tertiären organischen Basen mit einer Verbindung der allgemeinen Formel

$$Z - D - SO_m - R_1 \qquad (III),$$

in der

D, $R_1$ und m wie eingangs definiert sind und

Z eine nukleophil austauschbare Gruppe wie ein Halogenatom oder einen Sulfonsäureesterrest darstellt, umgesetzt wird oder

b) zur Herstellung von Verbindungen der allgemeinen Formel I, in der m die Zahl 1 oder 2 darstellt, eine Verbindung der allgemeinen Formel

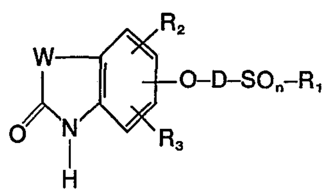 (IV),

in der

$R_1$ bis $R_3$, D und W wie eingangs definiert sind und n die Zahl 0 oder 1 darstellt, oxidiert wird oder

c) zur Herstellung einer Verbindung der allgemeinen Formel I, in der m die Zahl 0 darstellt, eine Verbindung der allgemeinen Formel

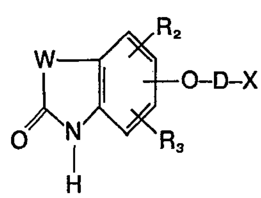 (V),

in der

$R_2$, $R_3$, D und W wie eingangs definiert sind, mit einer Verbindung der allgemeinen Formel

$$Y - R_1 \qquad (VI),$$

in der

$R_1$ wie eingangs definiert ist und

einer der Reste X oder Y in den Verbindungen der allgemeinen Formeln V und VI die Mercaptogruppe und der andere der Reste X oder Y eine nukleophil austauschbare Gruppe wie ein Halogenatom oder einen Sulfonsäureesterrest darstellt, umgesetzt wird oder

e) zur Herstellung eines Carbostyrils der allgemeinen Formel I, in der W die Vinylengruppe darstellt, ein 3,4-Dihydrocarbostyril der allgemeinen Formel

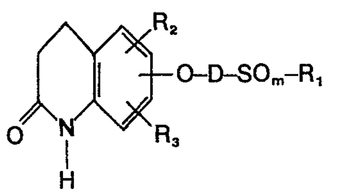 (VII) ,

in der

$R_1$ bis $R_3$, D und m wie eingangs definiert sind, dehydriert wird oder

f) zur Herstellung eines 3,4-Dihydro-carbostyrils der allgemeinen Formel I, in der W die Äthylengruppe und m die Zahl 0 oder 2 darstellen, ein Carbostyril der allgemeinen Formel

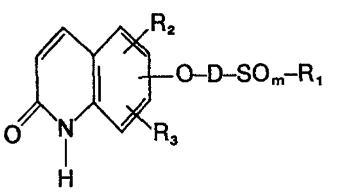 (VIII) ,

in der

$R_1$ bis $R_3$, D und m wie eingangs definiert sind, hydriert wird.

**Claims**

1. New carbostyril derivatives of general formula

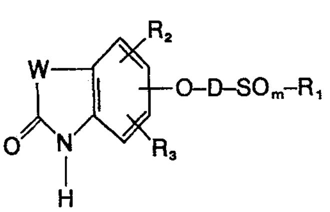 (I),

wherein

W represents a vinylene group (optionally substituted by a methyl group) or the ethylene group,

m represents the number 0, 1 or 2,

D represents a straight-chained or branched alkylene group with 2 to 6 carbon atoms, whilst there must be at least 2 carbon atoms between the oxygen atom and the $SO_m$ group, or a xylylene group,

$R_1$ represents a cycloalkyl group with 3 to 6 carbon atoms; an aryl group with 6 to 10 carbon atoms or an aralkyl group with 7 to 11 carbon atoms, whilst the above-mentioned aromatic nuclei may be monosubstituted by an alkyl group with 1 to 4 carbon atoms, by a hydroxy, methoxy, amino, acetylamino, nitro, carboxyl, cyclohexyl or phenyl group or by a halogen atom and, additionally, the above-mentioned monosubstituted phenyl groups may be mono- or disubstituted by alkyl groups with 1 to 4 carbon atoms and/or by halogen atoms (whilst the substituents of the phenyl nucleus may be identical or different); an N-methylcyclohexylamino-carbonylmethyl, amino-iminomethylene, pyridyl, pyridylmethyl, furfuryl, benzimidazolyl, benzothiazolyl, pyrimidyl, quinolyl, quinazolin-4-on-yl, 4,5-bis-(p-chlorophenyl)-oxazol-2-yl, pyridyloxide, triazolyl, methyl-pyridyl, methoxy-pyridyl, fluoropyridyl, chloropyridyl, aminopyridyl, acetylaminopyridyl or triphenyl-methyl group, or also a tert. butyl group, if m represents the number 1, and

$R_2$ and $R_3$, which may be identical or different, represent hydrogen or halogen atoms, alkyl groups with 1 to 4 carbon atoms, amino, acetylamino or nitro groups

2. New compounds of general formula I as claimed in claim 1, wherein

W, D and m are defined as in claim 1,

$R_1$ represents a cyclohexyl, benzyl, naphthyl, pyridyl, pyrimidyl, 1,2,4-triazolyl, pyridyl-oxide, furfuryl, triphenylmethyl, quinolyl, benzimidazolyl, benzothiazolyl, quinazolin-4-on-yl, 4,5-bis-(p-chlorophenyl)-oxazol-2-yl, N-methyl-cyclohexylamino-carbonylmethyl or aminoiminomethyl group, a phenyl group optionally substituted by a carboxyl, hydroxy, methoxy, amino, acetylamino, nitro, cyclohexyl or phenyl group, a phenyl group mono- or disubstituted by halogen atoms, a hydroxyphenyl, halophenyl or aminophenyl group substituted by two halogen atoms or by two alkyl groups with 1 to 4 carbon atoms,

$R_2$ represents a hydrogen, chlorine or bromine atom, the methyl, amino, acetylamino or nitro group, and

$R_3$ represents a hydrogen atom.

3. New compounds of general formula I as claimed in claim 1, wherein

W represents a vinylene group (optionally substituted by a methyl group) or the ethylene group,

m represents the number 0, 1 or 2,

D represents an alkylene group with 2 to 5 carbon atoms, whilst there must be at least 2 carbon atoms between the oxygen atom and the $SO_m$ group, $R_1$ represents a cyclohexyl, phenyl, benzyl, naphthyl, biphenylyl, cyclohexylphenyl, pyridyl, methylphenyl, methoxyphenyl, fluorophenyl, chlorophenyl, dichlorophenyl, trichlorophenyl, bromophenyl, dibromophenyl, bromomethyl-phenyl, aminodibromophenyl or hydroxy-di-tert.butyl-phenyl group, and

$R_2$ and $R_3$ each represent a hydrogen atom.

4. New compounds of general formula I as claimed in claim 1, wherein

W represents the ethylene, vinylene or 2-methylvinylene group,

m represents the number 1 or 2,

$R_2$ and $R_3$ each represent a hydrogen atom,

$R_1$ represents the cyclohexyl, phenyl, benzyl, naphthyl-(2), 2-methoxyphenyl, 4-chlorophenyl, 3,4-dichlorophenyl, 2,5-dichlorophenyl, 4-amino-3,5-dibromophenyl, 4-hydroxy-3,5-di-tert.butylphenyl or pyridyl-(2) group and

D represents the ethylene, n-propylene or n-butylene group.

5. 6-(4-Phenylsulphinylbutoxy)-3,4-dihydrocarbostyril.

6. 6-[4-(3,4-Dichlorophenylsulphinyl)-butoxy]-3,4-dihydrocarbostyril.

7. 6-[4-(2-Pyridylsulphonyl)-butoxy]-carbostyril.

8. Pharmaceutical compositions containing a compound as claimed in claims 1 to 7 together with one or more inert carriers or diluents.

9. Compounds as claimed in claims 1 to 7 for the preparation of a therapeutic agent for the treatment of thrombo-embolic diseases by a non-chemical method.

10. Process for the preparation of new carbostyril derivatives as claimed in claims 1 to 7, characterised in that

a) a hydroxy compound of general formula

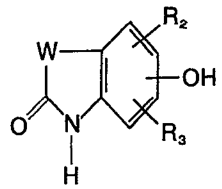 (II),

wherein

$R_2$, $R_3$ and W are as hereinbefore defined, or a salt thereof with inorganic or tertiary organic bases, is reacted with a compound of general formula

$$Z - D - SO_m - R_1 \quad (III),$$

wherein

D, $R_1$ and m are as hereinbefore defined and Z represents a nucleophilically exchangeable group such as a halogen atom or a sulphonic acid ester group, or

b) to prepare compounds of general formula I wherein m represents the number 1 or 2, a compound of general formula

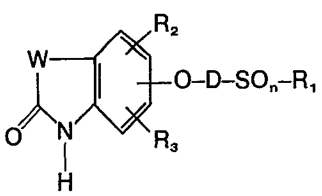 (IV),

wherein

$R_1$ to $R_3$, D and W are as hereinbefore defined and n represents the number 0 or 1, is oxidised, or

c) to prepare a compound of general formula I wherein m represents the number 0, a compound of general formula

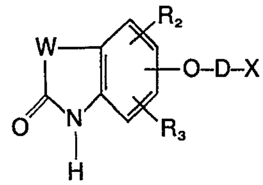 (V),

wherein

$R_2$, $R_3$, D and W are as hereinbefore defined, is reacted with a compound of general formula

$$Y - R_1 \quad (VI)$$

wherein

$R_1$ is as hereinbefore defined and one of the groups X or Y in the compounds of general formulae V and VI represents the mercapto group and the other group X or Y represents a nucleophilically exchangeable group such as a halogen atom or a sulphonic acid ester group, or

e) to prepare a carbostyril of general formula I wherein W represents the vinylene group, a 3,4-dihydro-carbostyril of general formula

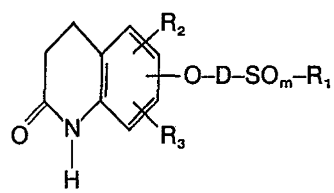 (VII) ,

wherein

$R_1$ to $R_3$, D and m are as hereinbefore defined, is dehydrogenated, or

f) to prepare a 3,4-dihydro-carbostyril of general formula I wherein W represents the ethylene group and m represents the number 0 or 2, a carbostyril of general formula

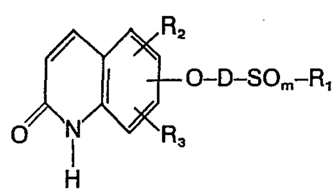 (VIII) ,

wherein

$R_1$ to $R_3$, D and m are as hereinbefore defined, is hydrogenated.

**Revendications**

1. Nouveaux dérivés de carbostyrile de formule générale:

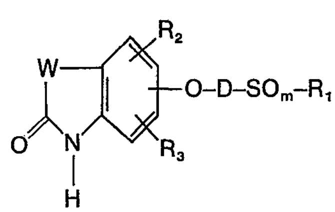 (I),

dans laquelle:

W représente un groupe vinylène éventuellement substitué par un groupe méthyle ou le groupe éthylène,

m représente le nombre 0, 1 ou 2,

D représents un groupe alcoylène rectiligne ou ramifié avec 2 à 6 atomes de carbone, au moins deux atomes de carbone devant se trouver entre l'atome d'oxygène et le groupe $SO_m$, ou un groupe xylylène,

$R_1$ représente un groupe cycloalcoyle avec 3 à 6 atomes de carbone, un groupe aryle avec 6 à 10 atomes de carbone ou un groupe aralcoyle avec 7 à 11 atomes de carbone, les noyaux aromatiques mentionnés ci-dessus étant monosubstitués par un groupe alcoyle avec 1 à 4 atomes de carbone, par un groupe hydroxy, méthoxy, amino, acétylamino, nitro, carboxyle, cyclohexyle, phényle ou un atome d'halogène et les groupes phényle monosubstitués mentionnés plus haut pouvant en outre être mono- ou disubstitués par des groupes alcoyle avec 1 à 4 atomes de carbone et/ou des atomes d'halogène (les substituants du noyau phényle pouvant être identiques ou différents), un groupe N-méthyl-cyclohexylamino-carbonylméthyle, amino-imino-méthylène, pyridyle, pyridylméthyle, furfuryle, benzimidazolyle, benzothiazolyle, pyrimidyle, quinoléyle, quinazoline-4-one-yle, 4,5-bis-(p-chlorophényl)-oxazol-2-yle, pyridyloxyde, triazolyle, méthyl-pyridyle, méthoxypyridyle,,fluoro-pyridyle, chloro-pyridyle, aminopyridyle, acétylamino-pyridyle ou triphénylméthyle ou également un groupe tert.butyle, lorsque m représente le nombre 1,

$R_2$ et $R_3$, qui peuvent être identiques ou différents, représentent des atomes d'hydrogène ou d'halogène, des groupes alcoyle avec 1 à 4 atomes de carbone, des groupes amino, acétylamino ou nitro.

2. Nouveaux composés de formule générale I selon la revendication 1, dans laquelle:

W, D et m sont définis comme à la revendication 1,

$R_1$ représente un groupe cyclohexyle, benzyle, naphtyle, pyridyle, pyrimidyle, 1,2,4-triazolyle, pyridyl-oxyde, furfuryle, triphénylméthyle, quinoléyle, benzimidazolyle, benzothiazolyle, quinazoline-4-one-yle, 4,5-bis-(p-chlorophényl)-oxazol-2-yle, N-méthyl-cyclohexylamino-carbonylméthyle ou aminoiminométhyle, un groupe phényle éventuellement substitué par un groupe carboxyle, hydroxy, méthoxy, amino, acétylamino, nitro, cyclohexyle ou phényle, un groupe phényle mono- ou disbustitué par des atomes d'halogène, un groupe hydroxyphényle, halogéno-phényle ou amino-phényle substitué par deux atomes d'halogène ou par deux groupes alcoyle avec 1 à 4 atomes de carbone,

$R_2$ représente un atome d'hydrogène, de chlore ou de brome, le groupe méthyle, amino, acétylamino ou nitro et

$R_3$ représente un atome d'hydrogène.

3. Nouveaux composés de formule générale I selon la revendication 1, dans laquelle:

W représente un groupe vinylène éventuellement substitué par un groupe méthyle ou le groupe éthylène,

m représente le nombre 0, 1 ou 2,

D représente un groupe alcoylène avec 2 à 5 atomes de carbone, au moins 2 atomes de carbone devant se trouver entre l'atome d'oxygène et le groupe $SO_m$,

$R_1$ représente un groupe cyclohexyle, phényle, benzyle, naphtyle, biphénylyle, cyclohexylphényle, pyridyle, méthylphényle, méthoxyphényle, fluorophényle, chlorophényle, dichlorophényle, trichlorophényle, bromophényle, dibromophényle, bromo-méthylphényle, amino-dibromophényle ou hydroxy-di-tert.butylphényle,

$R_2$ et $R_3$ représentent chacun un atome d'hydrogène.

4. Nouveaux composés de formule générale I selon la revendication 1, dans laquelle:

W représente le groupe éthylène, vinylène ou 2-méthylvinylène,

m représente le nombre 1 ou 2,

$R_2$ et $R_3$ représentent chacun un atome d'hydrogène,

$R_1$ représente le groupe cyclohexyle, phényle, benzyle, naphtyle-(2), 2-méthoxyphényle, 4-chlorophényle, 3,4-dichlorophényle, 2,5-dichlorophényle, 4-amino-3,5-dibromophényle, 4-hydroxy-3,5-di-tert.butylphényle ou pyridyle-(2) et

D représente le groupe éthylène, n-propylène ou n-butylène.

5. Le 6-(4-phénylsulfinylbutoxy)-3,4-dihydrocarbostyrile.

6. Le 6-[4-(3,4-dichlorophénylsulfinyl)-butoxy]-3,4-dihydrocarbostyrile.

7. Le 6-[4-(2-pyridylsulfonyl)-butoxy]-carbostyrile.

8. Médicament renfermant un composé selon les revendications 1 à 7 conjointement à un ou plusieurs excipients ou diluants inertes.

9. Composés selon les revendications 1 à 7 pour la préparation d'un remède contre les maladies thrombo-emboliques par voie non chimique.

10. Procédé pour la préparation des nouveaux dérivés de carbostyrile selon les revendications 1 à 7, caractérisé en ce que:

a) on fait réagir un composé hydroxy de formule générale:

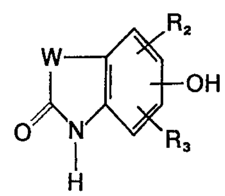

(II),

dans laquelle:

$R_2$, $R_3$ et W sont définis comme au début, ou leurs sels avec des bases minérales ou organiques tertiaires, avec un composé de formule générale:

$$Z - D - SO_m - R_1 \quad (III),$$

dans laquelle:

D, $R_1$ et m sont définis comme au début et

Z représente un groupe échangeable de façon

nucléophile tel qu'un atome d'halogène ou un reste d'acide sulfonique ou

b) pour préparer des composés de formule générale I dans laquelle m représente le nombre 1 ou 2, on oxyde un composé de formule générale:

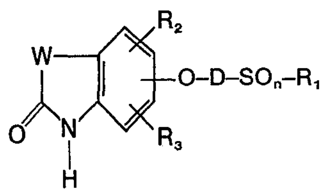

(IV),

dans laquelle:

$R_1$ à $R_3$, D et W sont définis comme au début et n représente le nombre 0 ou 1, ou

c) pour préparer un composé de formule générale I dans laquelle m représente le nombre 0, on fait réagir un composé de formule générale:

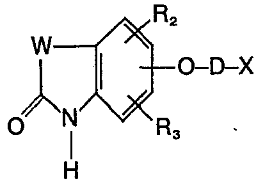

(V),

dans laquelle:

$R_2$, $R_3$, D et W sont définis comme au début, avec un composé de formule générale:

$$Y - R_1 \qquad (VI),$$

dans laquelle:

$R_1$ est défini comme au début et l'un des radicaux X ou Y dans les composés de formules générales V et VI représente le groupe mercapto et l'autre des radicaux X ou Y représente un groupe échangeable de façon nucléophile tel qu'un atome d'halogène ou un reste d'acide sulfonique ou

e) pour préparer un carbostyrile de formule générale I dans laquelle W représente le groupe vinylène, on déshydrogène un 3,4-dihydrocarbostyrile de formule générale:

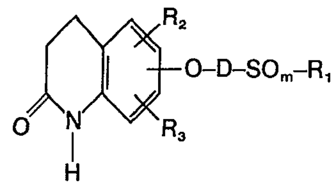

(VII) ,

dans laquelle:

$R_1$ à $R_3$, D et m sont définis comme au début, ou

f) pour préparer un 3,4-dihydrocarbostyrile de formule générale I dans laquelle W représente le groupe éthylène et m le nombre 0 ou 2, on hydrogène un carbostyrile de formule générale:

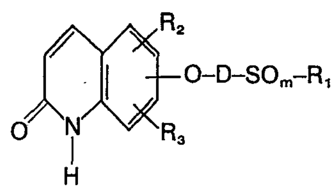

(VIII) ,

dans laquelle:

$R_1$ à $R_3$, D et m sont définis comme au début.